# EUROPEAN PATENT APPLICATION

(11) **EP 3 211 090 A1**
(43) Date of publication of application: **30.08.2017**
(21) Application number: 15852099.9
(22) Date of filing: 22.10.2015
(51) Int. Cl.: C12Q 1/68, G01N 33/15

(54) **METHOD FOR PREDICTING SENSITIVITY TO VASCULAR ENDOTHELIAL GROWTH FACTOR RECEPTOR INHIBITOR**

(30) Priority: 24.10.2014 KR 20140144762
(71) Applicant: Cbsbioscience Inc., Daejeon 305-343 (KR); Gil Medical Center, Incheon 21565 (KR)
(72) Inventor: PARK, Jin Young, Seoul 01721 (KR); MOON, Young Ho, Daejeon 34049 (KR); KWON, Jung-Hee, Daejeon 34016 (KR); KIM, Yun Soo, Seoul 06635 (KR); KIM, Ju Hyun, Incheon 22003 (KR); CHOI, Duck Joo, Seoul 03060 (KR)
(74) Representative: Awapatent AB
(86) International application number: PCT/KR2015/011238
(87) International publication number: WO 2016/064231

(57) **Abstract**

The present invention relates to a method for predicting sensitivity to treatment with vascular endothelial growth factor receptor (VEGFR) inhibitors, by using a biomarker. An effective means and anticancer therapy for cancer treatment of liver cancer are selected by predicting the effect of treatment with VEGFR inhibitors on liver cancer patients through the present invention, thereby increasing the treatment effect and minimizing the side effects of liver cancer treatment.

## Description

### [Technical Field]

The present invention relates to a method of predicting the sensitivity of a liver cancer patient to treatment with a vascular endothelial growth factor receptor inhibitor to determine whether or not the patient can receive a therapeutic effect from the treatment.

### [Background Art]

Cancer is one of the most fatal diseases that pose a threat to human health. In the USA alone, cancer affects nearly 1.3 million new patients each year, and is the second leading cause of death after heart disease, accounting for approximately 1 in 4 deaths. Although there have been significant advances in the medical treatment of certain cancers, the overall 5-year survival rate for all cancers has improved only by about 10% in the past 20 years. Furthermore, cancers, or malignant tumors, metastasize and grow rapidly in an uncontrolled manner, making timely detection and treatment extremely difficult.

Depending on the cancer type, patients typically have several treatment options available to them including chemotherapy, radiation and antibody-based drugs, but the therapeutic effects of these treatment options differ depending on the patient and the nature of cancer.

Particularly, liver cancer is a carcinoma having high resistance to anticancer therapy, and many kinds of molecular targeted drugs have been studied for use as chemotherapeutic agents, but only some of these drugs have been approved by the FDA or the like and marketed. In addition, even approved and commercially available anticancer drugs against liver cancer show very low therapeutic effects depending on the nature of cancer in cancer patients and the lesion of cancer. For example, Nexavar (Sorafenib), a new drug approved for the purpose of treating hepatocellular carcinoma, shows a low treatment rate of about 2-3%, and the side effects thereof are also very high. Namely, current guidelines for treatment of hepatocellular carcinoma are not based on the molecular characteristics of tumors, and thus there is a limitation in that the therapeutic effects of the guidelines cannot be guaranteed. Furthermore, although some genes known as biomarkers are present, a threshold value and diagnostic performance for predicting resistance or sensitivity to these genes have not been properly evaluated, and thus the clinical usefulness of these genes needs to be demonstrated through additional studies.

Therefore, there is a need for research and development on a specific biomarker making it possible to determine whether an individual patient will respond to any treatment, and on a threshold value and diagnostic performance for predicting resistance or sensitivity by use of the biomarker. In addition, there is a need to develop a method that makes it possible to select anticancer therapy and that can achieve anticancer treatment by effectively introducing an appropriate anticancer drug into a patient.

### [Disclosure]

### [Technical Problem]

The present invention is intended to provide a method for predicting sensitivity to a vascular endothelial growth factor receptor (VEGFR) inhibitor, the method comprising the steps of: measuring the expression levels of mTOR in samples isolated from liver cancer patients; and comparing the expression levels of mTOR with a reference level of mTOR expression, and selecting samples having an mTOR expression level equal to or higher than the reference level.

The present invention is also intended to provide a composition for predicting sensitivity to a vascular endothelial growth factor receptor (VEGFR) inhibitor, the composition containing an agent for measuring the mRNA expression level of mTOR.

The present invention is also intended to provide a kit for predicting sensitivity to a vascular endothelial growth factor receptor (VEGFR) inhibitor, the kit comprising the composition.

The present invention is also intended to provide a method of treating liver cancer using a vascular endothelial growth factor receptor (VEGFR) inhibitor, the method comprising the steps of: (a) measuring the expression levels of mTOR in samples isolated from liver cancer patients; (b) comparing the expression levels of mTOR with a reference level of mTOR expression; (b) selecting samples having an mTOR expression level equal to or higher than the reference level; and (c) administering a therapeutically effective amount of the vascular endothelial growth factor receptor (VEGFR) inhibitor to the liver cancer patients showing the mTOR expression level equal to or higher than the reference level.

### [Technical Solution]

The present inventors have made extensive efforts to select a patient having high sensitivity to a vascular endothelial growth factor receptor (VEGFR) inhibitor to thereby maximize the effect of anticancer treatment, and as a result, have found that, when the expression level of mTOR, VEGFR2, PDGFRβ, FGFR1, cKIT, EGFR or cRAF is measured, sensitivity to the vascular endothelial growth factor receptor (VEGFR) inhibitor can be predicted based on the expression level and treatment benefit score, thereby completing the present invention.

The present invention provides a method for predicting sensitivity to a vascular endothelial growth factor receptor (VEGFR) inhibitor, the method comprising the steps of: measuring the expression levels of mTOR in samples isolated from liver cancer patients; and comparing the expression levels of mTOR with a reference level of mTOR expression, and selecting samples having an mTOR expression level equal to or higher than the reference level.

In the present invention, the vascular endothelial growth factor receptor (VEGFR) inhibitor is a therapeutic agent that exhibits an anticancer effect by inhibiting vascular endothelial growth factor receptor (VEGFR), and it may be, for example, an anticancer agent, such as brivanib, sunitinib, linifanib, regorafenib, sorafenib or the like. According to one embodiment of the present invention, the vascular endothelial growth factor receptor (VEGFR) inhibitor is sorafenib.

As used herein, the term "sensitivity" refers to the property capable of benefiting or providing a therapeutic effect from treatment with a vascular endothelial growth factor receptor (VEGFR) inhibitor that is a targeted anticancer drug. For example, the term means that the tumor size is reduced by 5%, 10,% 15%, 20%, 25%, 30% or more after anticancer treatment. Preferably, the term means that the tumor size is reduced by 30% or more (complete response and partial response rates according to RECIST (Response Evaluation Criteria in Solid Tumors), criteria for evaluating the response of solid tumors to anticancer agents).

Namely, according to the present invention, sensitivity to a vascular endothelial growth factor receptor (VEGFR) inhibitor is predicted, thereby predicting the possibility of treatment with the vascular endothelial growth factor receptor (VEGFR) inhibitor and the prognosis of the treatment and selecting an effective means and anticancer therapy for anticancer treatment to thereby increase therapeutic effects and minimize the side effects of cancer treatment.

In particular, the present invention have advantages over conventional inventions in that it can exhibit the effect of sorafenib treatment, showing high accuracy by measuring the expression level of a biomarker of mTOR alone, and in that an increase in the mRNA level of mTOR shows high sensitivity to sorafenib treatment. Furthermore, the present invention shows the results of predicting a high level of sensitivity by examining sensitivity directly in patient samples, not in cell lines.

The method for predicting sensitivity to the vascular endothelial growth factor receptor (VEGFR) inhibitor according to the present invention comprises measuring a step of measuring the expression levels of mTOR in samples isolated from liver cancer patients.

As used herein, the term "liver cancer patients" refers to patients having primary liver cancer that is a malignant tumor occurring in the liver. Preferably, the term refers to patients having hepatocellular carcinoma among hepatocellular carcinoma and intrahepatic bile duct cancer, which are primary liver cancers. In addition, the samples include whole blood, plasma, serum, tissue or the like isolated from liver cancer patients. According to one embodiment of the present invention, liver tumor tissues are used. These samples may be suitably processed according to methods known in the art in order to measure expression levels.

In the present invention, "measuring the expression levels" includes measuring mRNA expression levels. In the present invention, "measuring mRNA expression levels" is a process of examining the presence and expression level of mRNA of a gene in samples in order to predict sensitivity to the vascular endothelial growth factor receptor (VEGFR) inhibitor, and may be performed by measuring the mRNA levels. Assay methods for this measurement include, but are not limited to, RT-PCR, competitive RT-PCR, real-time RT-PCR, RNase protection assay (RPA), Northern blotting, DNA chip assay, etc.

According to one embodiment of the present invention, the mRNA expression levels are measured by real-time RT-PCR. Furthermore, the mRNA expression level can be quantified as 2^{-ΔCt} by measuring the CT value of (the number of cycles required to reach the threshold value) of each gene and calculating the ΔCT value (CT of each marker minus the average CT value of a reference gene). In the present invention, the mTOR expression levels are measured in order to predict sensitivity to the vascular endothelial growth factor receptor (VEGFR) inhibitor. mTOR (mammalian target of rapamycin) is a serine/threonine kinase gene that is targeted by rapamycin in mammals, and has a nucleotide sequence of SEQ ID NO: 1.

As used herein, the term "reference level" refers to the same level as the expression level of genes capable of showing high diagnostic predictability detected and identified from reference samples by the method described herein. The reference level can be relative to a number or value derived from population studies, including subjects having the same cancer, subjects having the same or similar age range, subjects in the same or similar ethnic group, subjects having family histories of cancer, or relative to the starting sample of a subject undergoing treatment for cancer. Such reference levels can be derived from statistical analyses and/ or risk prediction data of populations obtained from mathematical algorithms and computed indices. Reference indices can also be constructed and used using algorithms and other methods of statistical and structural classification.

In certain embodiments, the term "reference level" herein refers to a predetermined value. For example, a person skilled in the art will appreciate that the reference level is predetermined and set to meet routine requirements in terms of specificity, sensitivity and/or accuracy. For example, sensitivity or specificity, respectively, has to be set to certain limits, e.g. 80%, 90% or 95%, respectively. These requirements may also be defined in terms of positive or negative predictive values. The reference level may be predetermined in reference samples from healthy individuals or predetermined from the disease entity to which the patient belongs.

In the present invention, a statistical analysis method such as mean value calculation or ROC curve analysis may be used to determine the reference level of expression.

ROC curve analysis according to one embodiment of the present invention is performed in terms of sensitivity, specificity and accuracy using a curve that shows the performance of diagnosis. In the present invention, sensitivity is the ratio of true positives to the sum of persons having sensitivity to a vascular endothelial growth factor receptor (VEGFR) inhibitor; specificity is the ratio of true negatives to the sum of persons having no sensitivity to a vascular endothelial growth factor receptor (VEGFR) inhibitor; and accuracy is the ratio of hits to all cases. When both specificity and sensitivity are high, the accuracy of test results increases. Thus, the x-axis in the ROC curve is 1-specificity, and the y-axis is sensitivity, and the AUC (area under curve) indicating accuracy means the area under the curve.

According to one embodiment of the present invention, "reference level" is a threshold value that shows a high predictive effect on the prediction of sensitivity to a vascular endothelial growth factor receptor (VEGFR) inhibitor. For the prediction of sensitivity, the expression level of mTOR, which corresponds to an AUC value higher than the average level (about 0.6), preferably the highest AUC value, in the ROC curve analysis of mTOR, is selected as the best threshold value (criterion), and a sample having a mTOR expression level equal to or higher than the reference level is predicted to have high sensitivity to a vascular endothelial growth factor receptor (VEGFR) inhibitor. For example, the reference level for the mRNA expression level according to one embodiment of the present invention may be 0.0007 (criterion), preferably 0.0004.

As used herein, the term "more than" or "higher than" means a level higher than the reference level or means an overall increase of 1%, 2%, 5%, 10%, 20%, 25%, 30%, 40%, 50%, 60%, 70%, 80%, 85%, 90%, 95%, 96%, 97%, 98%, 99% or more in the expression level detected by the method described herein, compared to the expression level in the reference sample. As used herein, the term "less than" or "lower than" means a level lower than the reference level or means an overall decrease of 1%, 2%, 5%, 10%, 20%, 25%, 30%, 40%, 50%, 60%, 70%, 80%, 85%, 90%, 95%, 96%, 97%, 98%, 99% or more in the expression level detected by the method described herein, compared to the expression level in the reference sample.

The method according to the present invention may further comprises the steps of: measuring the expression levels of one or more biomarkers, selected from among VEGFR2 and PDGFRβ, together with measurement of the expression level of mTOR; and comparing the expression level of each of the biomarkers with reference levels for the biomarkers together with comparing the mTOR expression level with the reference level, and selecting samples having expression levels equal to or higher than the reference levels of the biomarkers.

In the present invention, VEGFR2 (vascular endothelial growth factor receptor 2) is a tyrosine kinase-encoding gene acting as the receptor of vascular endothelial growth factor (VEGF) A, C, D and E, and has a nucleotide sequence of SEQ ID NO: 2.

In the present invention, PDGFRβ (platelet-derived growth factor receptor β) is a tyrosine kinase-encoding gene acting as the receptor of platelet-derived growth factor (PDGF), and has a nucleotide sequence of SEQ ID NO: 3.

Preferably, the biomarkers used together with mTOR in the method for predicting sensitivity are VEGFR2 and PDGFRβ.

In the method for predicting sensitivity, a treatment benefit score (TBS) can be calculated from the expression level of mTOR, and sensitivity to the vascular endothelial growth factor receptor (VEGFR) inhibitor can be predicted therefrom. Preferably, sensitivity to the vascular endothelial growth factor receptor (VEGFR) inhibitor can be predicted from a treatment benefit score (TBS) obtained by measuring the expression levels of mTOR and one or more selected from among VEGFR2 and PDGFRβ and calculating the sum of the expression levels. In the present invention, "treatment benefit score" is a value obtained from information indicating that a patient can receive a therapeutic effect from the vascular endothelial growth factor receptor (VEGFR) inhibitor. If a patient has a treatment benefit score equal to or higher than a certain score, determined based on the reference level, the patient has sensitivity to sorafenib. According to one embodiment of the present invention, the treatment benefit score can be calculated by multiplying the sum of mRNA expression levels, quantified as 2^{-ΔCt}, by 100. Herein, to determine the reference level, the AUC value in ROC curve analysis of the treatment benefit score, which is equal to higher than an average level (about 0.6), preferably the highest AUC value, is used as the best threshold value (criterion).

The present invention also provides a composition for predicting sensitivity to a vascular endothelial growth factor receptor (VEGFR) inhibitor, the composition containing an agent for measuring the mRNA expression level of mTOR.

The composition for predicting sensitivity to the vascular endothelial growth factor receptor (VEGFR) inhibitor makes it possible to predict sensitivity to the vascular endothelial growth factor receptor (VEGFR) inhibitor by measuring the mRNA expression level of mTOR.

Preferably, the composition for predicting sensitivity to the vascular endothelial growth factor receptor (VEGFR) inhibitor may further contain an agent for measuring the mRNA expression level of at least one biomarker selected from among VEGFR2 and PDGFRβ. More preferably, the composition for predicting sensitivity to the vascular endothelial growth factor receptor (VEGFR) inhibitor further contains agents for measuring the mRNA expression levels of VEGFR2 and PDGFRβ.

Preferably, the composition for predicting sensitivity to the vascular endothelial growth factor receptor (VEGFR) inhibitor may contain any one or more of a primer pair, a probe and an anti-sense nucleotide, which are agents for measuring mRNA expression levels and bind specifically to the genes. The sequence of the above-described primer pair, probe or antisense nucleotide can be easily designed by those skilled in the art from the nucleotide sequences provided in the present invention. According to one embodiment of the present invention, the agent for measuring the mRNA expression level comprises a primer pair and a probe, and may have the primer pair and probe sequences of mTOR, VEGFR2 and/or PDGFRβ shown in Table 1 below.

The present invention also provides a kit for predicting sensitivity to a vascular endothelial growth factor receptor (VEGFR) inhibitor, the kit containing a composition for predicting sensitivity to the vascular endothelial growth factor receptor (VEGFR) inhibitor.

The kit for predicting sensitivity to a vascular endothelial growth factor receptor (VEGFR) inhibitor may further contain an agent for measuring the mRNA expression level of at least one biomarker selected from among VEGFR2 and PDGFRβ. More preferably, the for predicting sensitivity to the vascular endothelial growth factor receptor (VEGFR) inhibitor further contains agents for measuring the mRNA expression levels of VEGFR2 and PDGFRβ.

The kit makes it possible to predict sensitivity to the vascular endothelial growth factor receptor (VEGFR) inhibitor by measuring the expression level of any one or more of polynucleotides encoding the proteins. The kit for predicting sensitivity to the vascular endothelial growth factor receptor (VEGFR) inhibitor according to the present invention may contain not only a primer pair or a probe for measuring expression levels, but also one or more other constituent compositions or devices suitable for analysis of the polynucleotides. Preferably, the diagnostic kit for quantitative detection of the above-described polynucleotides or genes according to the present invention may contain one or more oligonucleotides that bind specifically to the polynucleotides encoding the four proteins. Specifically, the diagnostic kit may contain primers corresponding to the oligonucleotides, reverse transcriptase, Taq polymerase, primers for PCR, and dNTP. To measure polynucleotide expression levels, a kit employing an analytical method described with respect to "the measurement of mRNA expression levels" may be used. For example, the kit may be selected from among RT-PCR kits, competitive RT-PCR kits, real-time RT-PCR kits, real-time RT-PCR kits, and DNA chip kits.

The present invention also provides a method of treating liver cancer using a vascular endothelial growth factor receptor (VEGFR) inhibitor, the method comprising the steps of:
(a) measuring the expression levels of mTOR in samples isolated from liver cancer patients; (b) comparing the expression levels of mTOR with a reference level of mTOR expression, and selecting samples having an mTOR expression level equal to or higher than the reference level; and (c) administering a therapeutically effective amount of the vascular endothelial growth factor receptor (VEGFR) inhibitor to the liver cancer patients showing the mTOR expression level equal to or higher than the reference level.

In addition, the method for treating liver cancer may further comprise: measuring the expression levels of mTOR and at least one selected from VEGFR2 and PDGFRβ; and calculating the sum of the expression levels, and selecting samples in which the sum of the expression levels is equal to or higher than a reference level for the sum of the expression levels. More preferably, the biomarkers, the levels of which are measured together with the level of mTOR and compared with the reference level, are VEGFR2 and PDGFRβ.

Based on the sum of the expression levels (preferably mRNA expression levels of mTOR and the biomarker, samples having a treatment benefit score equal to or higher than the reference level are selected. After selection of the samples, a therapeutically effective amount of the vascular endothelial growth factor receptor (VEGFR) inhibitor may be administered to a liver cancer patient showing the treatment benefit score equal to or higher than the reference level.

The vascular endothelial growth factor receptor (VEGFR) inhibitor is any one or more selected from among brivanib, sunitinib, linifanib, regorafenib, and sorafenib. Furthermore, the method may further comprise a step of administering a therapeutically effective amount of a therapeutic agent selected from the group consisting of cytotoxic agents, chemotherapeutic agents, growth inhibitors, antiangiogenic agents, and combinations thereof.

The present invention also provides the use of an agent for measuring the expression level of mTOR, for prediction of sensitivity to a vascular endothelial growth factor receptor (VEGFR) inhibitor. The use according to the present invention may further comprise, in addition to the use of the agent for measuring the expression level of mTOR, the use of at least one biomarker selected from VEGFR2 and PDGFRβ. Preferably, the agent comprises agents for measuring the expression levels of VEGFR2 and PDGFRβ.

The present invention also provides a method for predicting a vascular endothelial growth factor receptor (VEGFR) inhibitor, the method comprising the steps of: measuring the expression levels of FGFR1, cKIT, EGFR (epidermal growth factor receptor) or cRAF in samples isolated from liver cancer patients; and comparing the expression levels of FGFR1, cKIT, EGFR or cRAF with a reference level of expression of each of the these biomarkers, and selecting samples having an expression level higher than the reference level.

The present invention also provides a composition for predicting a vascular endothelial growth factor receptor (VEGFR) inhibitor, the composition containing an agent for measuring the mRNA expression level of FGFR1, cKIT, EGFR or cRAF.

The present invention also provides a kit for predicting a vascular endothelial growth factor receptor (VEGFR) inhibitor, the kit containing the composition for predicting the vascular endothelial growth factor receptor (VEGFR) inhibitor.

The present invention also provides a method for treating liver cancer using a vascular endothelial growth factor receptor (VEGFR) inhibitor, the method comprising the steps of:
(a) measuring the expression levels of FGFR1, cKIT, EGFR or cRAF in samples isolated from liver cancer patients; (b) comparing the expression levels of FGFR1, cKIT, EGFR or cRAF with a reference level of expression of each of the these biomarkers, and selecting samples having an expression level higher than the reference level; and (c) administering a therapeutically effective amount of the vascular endothelial growth factor receptor (VEGFR) inhibitor to the selected liver cancer patients.

The present invention also provides the use of an agent for measuring the expression level of FGFR1, cKIT, EGFR or cRAF, for predicting sensitivity to a vascular endothelial growth factor receptor (VEGFR) inhibitor.

In the present invention FGFR1 (fibroblast growth factor receptor 1) is a tyrosine kinase-encoding gene acting as the receptor of fibroblast growth factor (FGF), and has a nucleotide sequence of SEQ ID NO: 4.

In the present invention, cKIT is a tyrosine kinase-encoding gene acting as the receptor of stem cell factor (SCF), and has a nucleotide sequence of SEQ ID NO: 5.

In the present invention, EGFR (epidermal growth factor receptor) is a tyrosine kinase-encoding gene acting as the receptor of epidermal growth factor (EGF), and has a nucleotide sequence of SEQ ID NO: 6.

In the present invention, cRAF is a kinase-encoding gene belonging to the family of RAF serine/threonine kinases well-known as oncogenes, and has a nucleotide sequence of SEQ ID NO: 7.

The matters mentioned in the prediction method, composition, kit, treatment method and use of the present invention are applied in the same manner unless they are not contradictory to each other.

### [Advantageous Effects]

According to the present invention, effects on the treatment of liver cancer patients with a vascular endothelial growth factor receptor (VEGFR) inhibitor are predicted. Based on the prediction, an effective means and anticancer therapy for treatment of liver cancer are selected, thereby increasing treatment effects and minimizing the side effects of liver cancer treatment.

### [Description of Drawings]

FIG. 1 shows the results of analyzing the expression level distributions of mTOR, VEGFR2, PDGFRβ, FGFR1, cKIT, EGFR or cRAF in responders and non-responders to sorafenib.
FIG. 2 shows the results of analyzing the treatment benefit score distribution of responders to sorafenib and non-responders to sorafenib by use of mTOR, a combination of mTOR and VEGFR2, a combination of mTOR and PDGFRβ, or a combination of mTOR, VEGFR2 and PDGFRβ.
FIG. 3 shows the results of receiver operating characteristic (ROC) analysis performed to confirm the ability of treatment benefit scores to predict and classify responders to sorafenib and non-responders to sorafenib.

### [Mode for Invention]

The advantages and features of the present invention, and the way of attaining them, will become apparent with reference to the examples described below. However, the present invention is not limited to the examples disclosed below and can be embodied in a variety of different forms; rather, these examples are provided so that this disclosure will be thorough and complete, and will fully convey the scope of the present invention to those skilled in the art. The scope of the present invention will be defined by the appended claims.

### Example 1: RNA Extraction and cDNA Synthesis

From 31 liver cancer patients who were diagnosed as having liver cancer, received hepatectomy or liver transplantation, showed recurrence of liver cancer, and received treatment with Nexavar (Sorafenib) in Ajou University Medical Center, Korea University Anam Hospital and Keimyung University Dongsan Medical Center, liver cancer tissues were obtained under informed consent. In the following manner, RNA was extracted from each of the tissues and cDNA was synthesized therefrom.

Total RNA was extracted from liver cancer tissue and the surrounding normal tissue using an RNeasy Mini kit (Qiagen, Germany) according to the manufacturer's instructions. The extracted total RNA was quantified using Bioanalyzer 2100 (Agilent Technologies, USA). In the extraction step, the RNA extract was treated with DNase I to remove genomic DNA. Each sample comprising 4 µg of total RNA was incubated with 2 µl of 1 µM oligo d(T)18 primer (Genotech, Korea) at 70°C for 7 minutes and cooled on ice for 5 minutes. A total of 11 µl of an enzyme mixture [2 µl of 0.1 M DTT (Duchefa, the Netherlands), 2 µl of 10X reverse transcription buffer, 5 µl of 2 mM dNTP, 1 µl of 200 U/µl MMLV reverse transcriptase and 1 µl of 40 U/µl RNase inhibitor (Enzynomics, Korea)] was separately prepared. The enzyme mixture was added to the mixture containing the RNA, and was incubated at 42°C for 90 minutes, and then incubated at 80°C for 10 minutes to inactivate the reverse transcriptase. Diethyl pyrocarbonate (DEPC)-treated water was added to the mixture to a final volume of 400 µl.

### Example 2: Quantitative Real-Time PCR

In order to measure the mRNA expression level of mTOR, VEGFR2, PDGFRβ, FGFR1, cKIT, EGFR or cRAF, for each of the cDNA samples obtained in Example 1, the gene markers shown in Table 1 below were amplified by real-time PCR using PRISM 7900HT (Applied Biosystems, USA) according to the manufacturer's instructions.

Real-time PCR analysis was performed using a total of 10 µl of a volume consisting of 5 µl of 2x TaqMan gene expression master mix (Applied Biosystems, USA), 1 µl of each of 5 µM forward and reverse primers, 1 µl of 1 µM probe (Genotech, Korea) and 2 µl of cDNA (the same amount of water as control) . The PCR amplification was performed by denaturation at 95°C for 10 minutes, followed by cycle reactions consisting of denaturation at 95°C for 15 sec and synthesis at 60°C for 1 minute. The primer and probe sequences were designed using Primer Express 3.0 (Applied Biosystems, USA), and all the probe sequences were labeled with FAM at the 5' end and TAMRA at the 3' end. For each of the markers, the primer and probe sequences shown in Table 1 below were used.

**Table 1: Information of primer and probe sequences for marker genes**

| **Genes** | **Sequences** | |
|---|---|---|
| VEGFR2 | F | CACCACTCAAACGCTGACATGTA (SEQ ID NO: 8) |
| | R | CCAACTGCCAATACCAGTGGAT (SEQ ID NO: 9) |
| | P | TATGCCATTCCTCCCCCGCATCA (SEQ ID NO: 10) |
| PDGFR-β | F | AGCGCTGGCGAAATCG (SEQ ID NO: 11) |
| | R | TTCACGCGAACCAGTGTCA (SEQ ID NO: 12) |
| | P | CTGTCCACGCGCAACGTGTCG (SEQ ID NO: 13) |
| C-KIT | F | CAGATTTCAGAGAGCACCAATCA (SEQ ID NO: 14) |
| | R | AATGGTCTACCACGGGCTTCT (SEQ ID NO: 15) |
| | P | TTACTCCAACTTAGCAAACTGCAGCCCCAA (SEQ ID NO: 16) |
| C-RAF | F | GAGGTCGACATCCACACCTAATG (SEQ ID NO: 17) |
| | R | TCGAATTGCATCCTCAATCATC (SEQ ID NO: 18) |
| | P | CCACATGGTCAGCACCACCCTGC (SEQ ID NO: 19) |
| EGFR | F | GAAGGAGCTGCCCATGAGAA (SEQ ID NO: 20) |
| | R | GACTATGTCCCGCCACTGGAT (SEQ ID NO: 21) |
| | P | AAATCCTGCATGGCGCCGTGC (SEQ ID NO: 22) |
| mTOR | F | AGGCCGCATTGTCTCTATCAA (SEQ ID NO: 23) |
| | R | GCAGTAAATGCAGGTAGTCATCCA (SEQ ID NO: 24) |
| | P | TGCAATCCAGCTGTTTGGCGCC (SEQ ID NO: 25) |
| FGFR1 | F | CACGGGACATTCACCACATC (SEQ ID NO: 26) |
| | R | GGGTGCCATCCACTTCACA (SEQ ID NO: 27) |
| | P | ACTATAAAAAGACAACCAACGGCCGACTGC (SEQ ID NO: 28) |

The expression of each marker gene was repeatedly measured three times, and normalized to the average expression of five reference genes (B2M, GAPDH, HMBS, HPRT1, and SDHA). For the reference genes, the primer and probe sequences shown in Table 2 below were used.

**Table 2: Information of primer and probe sequences for reference genes**

| **Genes** | **Sequences** | |
|---|---|---|
| B2M | F | CATTCGGGCCGAGATGTCT (SEQ ID NO: 29) |
| | R | CTCCAGGCCAGAAAGAGAGAGTAG (SEQ ID NO: 30) |
| | P | CCGTGGCCTTAGCTGTGCTCGC (SEQ ID NO: 31) |
| GAPDH | F | CACATGGCCTCCAAGGAGTAA (SEQ ID NO: 32) |
| | R | TGAGGGTCTCTCTCTTCCTCTTGT (SEQ ID NO: 33) |
| | P | CTGGACCACCAGCCCCAGCAAG (SEQ ID NO: 34) |
| HMBS | F | CCAGGGATTTGCCTCACCTT (SEQ ID NO: 35) |
| | R | AAAGAGATGAAGCCCCCACAT (SEQ ID NO: 36) |
| | P | CCTTGATGACTGCCTTGCCTCCTCAG (SEQ ID NO: 37) |
| HPRT1 | F | GCTCGAGATGTGATGAAGGAGAT (SEQ ID NO: 38) |
| | R | CCAGCAGGTCAGCAAAGAATT (SEQ ID NO: 39) |
| | P | CCATCACATTGTAGCCCTCTGTGTGCTC (SEQ ID NO: 40) |
| SDHA | F | CACCTAGTGGCTGGGAGCTT (SEQ ID NO: 41) |
| | R | GCCCAGTTTTATCATCTCACAAGA (SEQ ID NO: 42) |
| | P | TGGCACTTACCTTTGTCCCTTGCTTCA (SEQ ID NO: 43) |

The CT value (the number of cycles required to reach a threshold value) of each marker was measured, and the ΔCT value (the CT value of each marker minus the mean CT value of reference genes) was calculated, thereby the mRNA expression level of each marker was expressed as 2^{-ΔCt}.

### Example 3: Statistical Analysis

Using the expression levels (2^{-ΔCt}) of the markers obtained in Example 2, a total of four statistical analyses (i.e., individual gene expression distribution analysis, ROC curve analysis, treatment benefit score analysis, and ROC curve analysis of treatment benefit scores) were performed in the following manner.

### (1) Analysis of Expression Distributions of Individual Genes

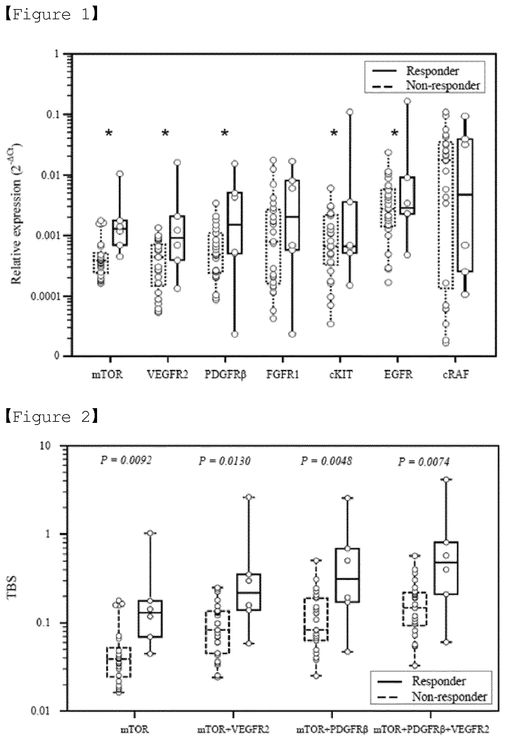

In order to examine the correlation between the therapeutic effect of sorafenib and the expression levels (2^{-ΔCT}) of the individual markers, the expression levels of the seven individual markers and the distribution thereof were analyzed for responders to sorafenib and non-responders to sorafenib, respectively, and the results of the analysis are shown in FIG. 1.

FIG. 1 shows the results of analyzing the mRNA expression levels of seven genes (i.e., mTOR, VEGFR2, PDGFRβ, FGFR1, cKIT, EGFR and cRAF) for responders and non-responders.

When expression of each of these markers showed that the expression level of mTOR was up-regulated in the responders (mean value of 2^{-ΔCT}: 0.0026) compared to in the non-responders (mean value of 2^{-ΔCT}: 0.0003), with statistical significance (P=0.0092). Furthermore, it was shown that the expression level of VEGFR2 was up-regulated in the responders (mean value of 2^{-ΔCT}: 0.0034) compared to in the non-responders (mean value of 2^{-ΔCT}: 0.0001), with statistical significance (P=0.0181) . It was also shown that the expression level of PDGFRβ was up-regulated in the responders (mean value of 2^{-ΔCT}: 0.0043) compared to in the non-responders (mean value of 2^{-ΔCT}: 0.0001), with statistical significance (P=0.0043). Moreover, it was shown that the expression level of FGFR1 was up-regulated in the responders (mean value of 2^{-ΔCT}: 0.0053) compared to in the non-responders (mean value of 2^{-ΔCT}: 0.0002).

In addition, it was shown that the expression level of cKIT was up-regulated in the responders (mean value of 2^{-ΔCT}: 0.0192) compared to in the non-responders (mean value of 2^{-ΔCT}: 0.0005), with statistical significance (P=0.0408). Furthermore, it was shown that the expression level of EGFR was up-regulated in the responders (mean value of 2^{-ΔCT}: 0.0302) compared to in the non-responders (mean value of 2^{-ΔCT}: 0.0022), with statistical significance (P=0.0495). It was also shown that the expression level of cRAF was down-regulated in the responders (mean value of 2^{-ΔCT}: 0.0276) compared to in the non-responders (mean value of 2^{-ΔCT}: 0.0310), with statistical significance (P=0.8089).

### (2) ROC curve analysis

In order to confirm whether the expression levels of individual markers in the 31 patients, calculated as described above, have the ability to predict and classify responders to sorafenib and non-responders to sorafenib, ROC (receiver operating characteristic) curve analysis was performed using various cut-off values.

Furthermore, based on the gene expression level corresponding to the average AUC value (about 0.6) or the highest AUC value in the ROC curve, the best threshold value (criterion) was obtained, and sensitivity, specificity, AUC value and p value, which correspond to each classifier and criterion, were obtained. The results of the analysis are shown in Table 3 below.

**Table 3: Sensitivity, specificity and accuracy for prediction of the response to sorafenib treatment of each gene**

| **Genes** | **Criterion** | **Sensitivity (%)** | **Specificity (%)** | **AUC** | **P value** |
|---|---|---|---|---|---|
| mTOR | >0.0007 | 66.67 | 84.0 | 0.65 | 0.041 |
| | >0.0004 | 100.0 | 72.0 | 0.860 | <0.0001 |
| VEGFR2 | >0.0004 | 66.67 | 48.0 | 0.57 | 0.082 |
| | >0.001 | 50.0 | 96.0 | 0.740 | 0.0761 |
| PDGFRβ | >0.0005 | 83.33 | 48.0 | 0.63 | 0.261 |
| | >0.0035 | 50.0 | 100.0 | 0.683 | 0.2723 |
| FGFR1 | >0.0006 | 66.67 | 44.0 | 0.55 | 0.630 |
| | >0.0042 | 50.0 | 88.0 | 0.607 | 0.510 |
| cKIT | >0.0007 | 50.0 | 48.00 | 0.511 | 0.54 |
| | >0.0031 | 33.3 | 96.0 | 0.587 | 0.559 |
| EGFR | >0.0015 | 83.33 | 28.0 | 0.55 | 0.539 |
| | >0.0022 | 83.3 | 48.0 | 0.587 | 0.5346 |
| cRaf | >0.0003 | 66.67 | 28.0 | 0.514 | 0.719 |
| | >0.0001 | 100.0 | 24.0 | 0.533 | 0.810 |

Table 3 above shows the results of ROC curve analysis performed to predict a response to sorafenib based on the mRNA expression level of each marker gene. The results of the ROC curve analysis indicated that the highest AUC was 0.860 (p *<* 0.0001) in mTOR. Particularly, it was shown that the AUC value was higher in mTOR, VEGRF2 and PDGFRβ than in other markers.

### (3) Calculation of Treatment Benefit Score

In order to examine the correlation between the therapeutic effect of sorafenib and treatment benefit scores based on the expression levels of mTOR, VEGFR2 and/or PDGFRβ, the treatment benefit scores were calculated.

The treatment benefit score was calculated by selecting and combining the markers having high AUC values as described above, calculating the sum of the expression levels of the selected markers, and multiplying the sum by 100.

The results of calculating the treatment benefit scores based on a combination of mTOR, VEGRF2 and PDGFRβ, which showed high AUC values, are shown in FIG. 2 for responders and non-responders.

As shown in FIG. 2, the treatment benefit score in the case of mTOR was up-regulated in the responders (mean value of treatment benefit scores (TBS): 0.03) compared to in the non-responders (mean value of treatment benefit scores (TBS): 0.26), with statistical significance (P=0.0092). Furthermore, it was shown that the treatment benefit score obtained using a combination of mTOR and VEGRF2 was up-regulated in the responders (mean value of treatment benefit scores (TBS): 0.61) compared to in the non-responders (mean value of treatment benefit scores (TBS): 0.04), with statistical significance (P=0.0130). It was also shown that the treatment benefit score obtained using a combination of mTOR and PDGFRβ was up-regulated in the responders (mean value of treatment benefit scores (TBS): 0.69) compared to in the non-responders (mean value of treatment benefit scores (TBS): 0.05), with statistical significance (P=0.0048). In addition, it was shown that the treatment benefit score obtained using a combination of mTOR, VEGRF2 and PDGFRβ was up-regulated in the responders (mean value of treatment benefit scores (TBS): 1.04) compared to in the non-responders (mean value of treatment benefit scores (TBS): 0.05), with statistical significance (P=0.0074).

### (4) ROC Curve Analysis of Treatment Benefit Scores

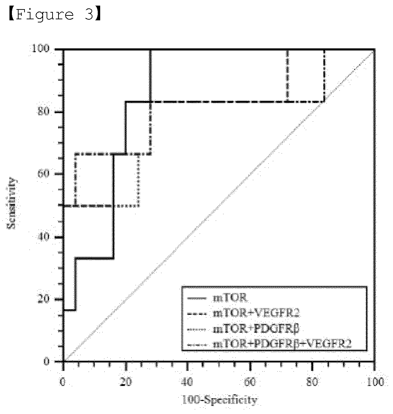

In order to evaluate the ability of the above-described treatment benefit scores to predict the therapeutic effect of sorafenib, ROC (receiver operating characteristic) analysis was performed using various cut-off values. Furthermore, based on the gene expression level corresponding to the average AUC value (about 0.6) or the highest AUC value in the ROC curve, the best threshold value (criterion) was obtained, and sensitivity, specificity, AUC value and p value, which correspond to each classifier and criterion, were obtained. The results of the analysis are shown in FIG. 3 and Table 4 below.

**Table 4: Sensitivity, specificity and accuracy of prediction of sorafenib treatment response, obtained based on treatment benefit scores**

| **Genes** | **Criterion** | **Sensitivity (%)** | **Specificity (%)** | **AUC** | **P value** |
|---|---|---|---|---|---|
| mTOR | >0.07 | 66.67 | 84.0 | 0.65 | 0.041 |
| | >0.04 | 100.00 | 72.0 | 0.860 | <0.0001 |
| mTOR+VEGFR2 | >0.18 | 50.0 | 88.0 | 0.61 | 0.028 |
| | >0.14 | 83.33 | 80.0 | 0.820 | 0.0070 |
| mTOR+PDGFRβ | >0.19 | 50.0 | 76.0 | 0.62 | 0.0312 |
| | >0.15 | 83.33 | 72.0 | 0.773 | 0.0472 |
| mTOR+VEGFR2+PDGFRβ | >0.15 | 83.3 | 48.0 | 0.643 | 0.0208 |
| | >0.4 | 66.7 | 96.0 | 0.807 | 0.0274 |

FIG. 3 and Table 4 above show the ROC curve based on the treatment benefit score obtained using mTOR alone or in combination with VEGRF2 and/or PDGFRβ. In the results of the ROC analysis based on the treatment benefit score obtained using mTOR alone or in combination with VEGRF2 and/or PDGFRβ, the AUC values were 0.860 when mTOR was used alone, 0.820 when mTOR was used in combination with VEGRF2, 0.773 when mTOR was used in combination with PDGFRβ, and 0.807 when mTOR was used in combination with VEGRF2 and PDGFRβ.

As can be seen from the foregoing, the markers and prediction method of the present invention show significant effects on prediction of the therapeutic effect of sorafenib. In particular, the therapeutic effect of sorafenib can be more accurately predicted from the treatment benefit scores obtained from mTOR and combinations of mTOR and additional markers.

## Claims

1. A method for predicting sensitivity to a vascular endothelial growth factor receptor (VEGFR) inhibitor, the method comprising the steps of:
(a) measuring expression levels of mTOR in samples isolated from liver cancer patients; and
(b) comparing the expression levels of mTOR with a reference level of mTOR expression, and selecting samples having the expression level of mTOR equal to or higher than the reference level.

2. The method of claim 1, wherein the step (a) further comprises measuring expression levels of one or more biomarkers selected from among VEGFR2 and PDGFRβ; and
the step (b) further comprises comparing the expression levels of the one or more biomarkers selected from among VEGFR2 and PDGFRβ, with a reference level of expression of each of the biomarkers, and selecting samples having the biomarker expression level equal to or higher than the reference level.

3. The method of claim 1 or 2, wherein the vascular endothelial growth factor receptor (VEGFR) inhibitor is any one or more selected from among brivanib, sunitinib, linifanib, regorafenib, and sorafenib.

4. The method of claim 1 or 2, wherein the step (a) of measuring expression levels is measuring mRNA expression levels.

5. The method of claim 4, wherein the step (a) of measuring mRNA expression levels is measuring mRNA expression levels of mTOR and one or more biomarkers selected from among VEGFR2 and PDGFRβ.

6. The method of claim 1 or 2, wherein the reference level is 0.6 or more as an AUC value.

7. The method of claim 1, wherein the reference level is 0.0007 or more based on the mRNA expression level of mTOR.

8. The method of claim 1 or 2, wherein the mTOR has a nucleotide sequence of SEQ ID NO: 1.

9. A composition for predicting sensitivity to a vascular endothelial growth factor receptor (VEGFR) inhibitor, the composition containing an agent for measuring the mRNA expression level of mTOR.

10. The composition of claim 9, wherein the composition further contains an agent for measuring mRNA expression levels of one or more biomarkers selected from among VEGFR2 and PDGFRβ.

11. The composition of claim 9, wherein the vascular endothelial growth factor receptor (VEGFR) inhibitor is any one or more selected from among brivanib, sunitinib, linifanib, regorafenib, and sorafenib.

12. The composition of claim 9, wherein the agent for measuring the mRNA expression level is a primer pair, a probe or an antisense nucleotide, which binds specifically to the genes.

13. A kit for predicting sensitivity to a vascular endothelial growth factor receptor (VEGFR) inhibitor, the kit containing the composition of any one of claims 9 to 12.

14. The kit of claim 13, wherein the kit is an RT-PCR kit, a competitive RT-PCR kit, a real-time RT-PCR kit, or a DNA chip kit.

15. A method of treating liver cancer using a vascular endothelial growth factor receptor (VEGFR) inhibitor, the method comprising the steps of:
(a) measuring expression levels of mTOR in samples isolated from liver cancer patients; (b) comparing the expression levels of mTOR with a reference level of mTOR expression, and selecting samples having an mTOR expression level equal to or higher than the reference level; and (c) administering a therapeutically effective amount of the vascular endothelial growth factor receptor (VEGFR) inhibitor to the liver cancer patients showing the mTOR expression level equal to or higher than the reference level.

16. Use of an agent for measuring mRNA expression level of mTOR, for prediction of sensitivity to a vascular endothelial growth factor receptor (VEGFR) inhibitor.


<110>     CBSBioScience
          GIL MEDICAL CENTER

<120>     METHOD OF PREDICTING SENSITIVITY FOR VASCULAR ENDOTHELIAL GROWTH
          FACTOR RECEPTOR INHIBITOR

<130>     P15025-CBS

<150>     KR 2014-0144762
<151>     2014-10-24

<160>     43

<170>     KopatentIn 2.0

<210>     1
<211>     8733
<212>     RNA
<213>     Homo sapiens

<220>
<221>     mRNA
<222>     (1)..(8733)
<223>     mechanistic target of rapamycin (serine/threonine kinase) (mTOR)

<400>     1
gctcccggct tagaggacag cggggaaggc gggcggtggg gcagggggcc tgaagcggcg        60

gtaccggtgc tggcggcggc agctgaggcc ttggccgaag ccgcgcgaac ctcagggcaa       120

gatgcttgga accggacctg ccgccgccac caccgctgcc accacatcta gcaatgtgag       180

cgtcctgcag cagtttgcca gtggcctaaa gagccggaat gaggaaacca gggccaaagc       240

cgccaaggag ctccagcact atgtcaccat ggaactccga gagatgagtc aagaggagtc       300

tactcgcttc tatgaccaac tgaaccatca catttttgaa ttggtttcca gctcagatgc       360

caatgagagg aaaggtggca tcttggccat agctagcctc ataggagtgg aaggtgggaa       420

tgccacccga attggcagat ttgccaacta tcttcggaac ctcctcccct ccaatgaccc       480

agttgtcatg gaaatggcat ccaaggccat tggccgtctt gccatggcag gggacacttt       540

taccgctgag tacgtggaat ttgaggtgaa gcgagccctg gaatggctgg gtgctgaccg       600

caatgagggc cggagacatg cagctgtcct ggttctccgt gagctggcca tcagcgtccc       660

taccttcttc ttccagcaag tgcaaccctt ctttgacaac atttttgtgg ccgtgtggga       720

ccccaaacag gccatccgtg agggagctgt agccgccctt cgtgcctgtc tgattctcac       780

aacccagcgt gagccgaagg agatgcagaa gcctcagtgg tacaggcaca catttgaaga       840

agcagagaag ggatttgatg agaccttggc caaagagaag ggcatgaatc gggatgatcg       900

gatccatgga gccttgttga tccttaacga gctggtccga atcagcagca tggagggaga       960

gcgtctgaga gaagaaatgg aagaaatcac acagcagcag ctggtacacg acaagtactg      1020

caaagatctc atgggcttcg gaacaaaacc tcgtcacatt acccccttca ccagtttcca      1080

ggctgtacag ccccagcagt caaatgcctt ggtggggctg ctggggtaca gctctcacca 1140

aggcctcatg ggatttggga cctcccccag tccagctaag tccaccctgg tggagagccg 1200

gtgttgcaga gacttgatgg aggagaaatt tgatcaggtg tgccagtggg tgctgaaatg 1260

caggaatagc aagaactcgc tgatccaaat gacaatcctt aatttgttgc cccgcttggc 1320

tgcattccga ccttctgcct tcacagatac ccagtatctc caagatacca tgaaccatgt 1380

cctaagctgt gtcaagaagg agaaggaacg tacagcggcc ttccaagccc tggggctact 1440

ttctgtggct gtgaggtctg agtttaaggt ctatttgcct cgcgtgctgg acatcatccg 1500

agcggccctg cccccaaagg acttcgccca taagaggcag aaggcaatgc aggtggatgc 1560

cacagtcttc acttgcatca gcatgctggc tcgagcaatg gggccaggca tccagcagga 1620

tatcaaggag ctgctggagc ccatgctggc agtgggacta agccctgccc tcactgcagt 1680

gctctacgac ctgagccgtc agattccaca gctaaagaag gacattcaag atgggctact 1740

gaaaatgctg tccctggtcc ttatgcacaa accccttcgc cacccaggca tgcccaaggg 1800

cctggcccat cagctggcct ctcctggcct cacgaccctc cctgaggcca gcgatgtggg 1860

cagcatcact cttgccctcc gaacgcttgg cagctttgaa tttgaaggcc actctctgac 1920

ccaatttgtt cgccactgtg cggatcattt cctgaacagt gagcacaagg agatccgcat 1980

ggaggctgcc cgcacctgct cccgcctgct cacaccctcc atccacctca tcagtggcca 2040

tgctcatgtg gttagccaga ccgcagtgca agtggtggca gatgtgctta gcaaactgct 2100

cgtagttggg ataacagatc ctgaccctga cattcgctac tgtgtcttgg cgtccctgga 2160

cgagcgcttt gatgcacacc tggcccaggc ggagaacttg caggccttgt ttgtggctct 2220

gaatgaccag gtgtttgaga tccgggagct ggccatctgc actgtgggcc gactcagtag 2280

catgaaccct gcctttgtca tgcctttcct gcgcaagatg ctcatccaga ttttgacaga 2340

gttggagcac agtgggattg gaagaatcaa agagcagagt gcccgcatgc tggggcacct 2400

ggtctccaat gccccccgac tcatccgccc ctacatggag cctattctga aggcattaat 2460

tttgaaactg aaagatccag accctgatcc aaacccaggt gtgatcaata atgtcctggc 2520

aacaatagga gaattggcac aggttagtgg cctggaaatg aggaaatggg ttgatgaact 2580

ttttattatc atcatggaca tgctccagga ttcctctttg ttggccaaaa ggcaggtggc 2640

tctgtggacc ctgggacagt tggtggccag cactggctat gtagtagagc cctacaggaa 2700

gtaccctact ttgcttgagg tgctactgaa ttttctgaag actgagcaga accagggtac 2760

acgcagagag gccatccgtg tgttagggct tttaggggct ttggatcctt acaagcacaa 2820

agtgaacatt ggcatgatag accagtcccg ggatgcctct gctgtcagcc tgtcagaatc 2880

caagtcaagt caggattcct ctgactatag cactagtgaa atgctggtca acatgggaaa 2940

cttgcctctg gatgagttct acccagctgt gtccatggtg gccctgatgc ggatcttccg 3000

agaccagtca ctctctcatc atcacaccat ggttgtccag gccatcacct tcatcttcaa 3060

gtccctggga ctcaaatgtg tgcagttcct gccccaggtc atgcccacgt tccttaacgt 3120

cattcgagtc tgtgatgggg ccatccggga atttttgttc cagcagctgg gaatgttggt 3180

gtcctttgtg aagagccaca tcagacctta tatggatgaa atagtcaccc tcatgagaga 3240

attctgggtc atgaacacct caattcagag cacgatcatt cttctcattg agcaaattgt 3300

ggtagctctt gggggtgaat ttaagctcta cctgccccag ctgatcccac acatgctgcg 3360

tgtcttcatg catgacaaca gcccaggccg cattgtctct atcaagttac tggctgcaat 3420

ccagctgttt ggcgccaacc tggatgacta cctgcattta ctgctgcctc ctattgttaa 3480

gttgtttgat gcccctgaag ctccactgcc atctcgaaag gcagcgctag agactgtgga 3540

ccgcctgacg gagtccctgg atttcactga ctatgcctcc cggatcattc accctattgt 3600

tcgaacactg gaccagagcc cagaactgcg ctccacagcc atggacacgc tgtcttcact 3660

tgtttttcag ctggggaaga agtaccaaat tttcattcca atggtgaata aagttctggt 3720

gcgacaccga atcaatcatc agcgctatga tgtgctcatc tgcagaattg tcaagggata 3780

cacacttgct gatgaagagg aggatccttt gatttaccag catcggatgc ttaggagtgg 3840

ccaaggggat gcattggcta gtggaccagt ggaaacagga cccatgaaga aactgcacgt 3900

cagcaccatc aacctccaaa aggcctgggg cgctgccagg agggtctcca aagatgactg 3960

gctggaatgg ctgagacggc tgagcctgga gctgctgaag gactcatcat cgccctccct 4020

gcgctcctgc tgggccctgg cacaggccta caacccgatg gccagggatc tcttcaatgc 4080

tgcatttgtg tcctgctggt ctgaactgaa tgaagatcaa caggatgagc tcatcagaag 4140

catcgagttg gccctcacct cacaagacat cgctgaagtc acacagaccc tcttaaactt 4200

ggctgaattc atggaacaca gtgacaaggg ccccctgcca ctgagagatg acaatggcat 4260

tgttctgctg ggtgagagag ctgccaagtg ccgagcatat gccaaagcac tacactacaa 4320

agaactggag ttccagaaag gccccacccc tgccattcta gaatctctca tcagcattaa 4380

taataagcta cagcagccgg aggcagcggc cggagtgtta gaatatgcca tgaaacactt 4440

tggagagctg gagatccagg ctacctggta tgagaaactg cacgagtggg aggatgccct 4500

tgtggcctat gacaagaaaa tggacaccaa caaggacgac ccagagctga tgctgggccg 4560

catgcgctgc ctcgaggcct tgggggaatg gggtcaactc caccagcagt gctgtgaaaa 4620

gtggaccctg gttaatgatg agacccaagc caagatggcc cggatggctg ctgcagctgc 4680

atggggttta ggtcagtggg acagcatgga agaatacacc tgtatgatcc ctcgggacac 4740

ccatgatggg gcattttata gagctgtgct ggcactgcat caggacctct tctccttggc 4800

acaacagtgc attgacaagg ccagggacct gctggatgct gaattaactg cgatggcagg 4860

agagagttac agtcgggcat atggggccat ggtttcttgc cacatgctgt ccgagctgga 4920

ggaggttatc cagtacaaac ttgtccccga gcgacgagag atcatccgcc agatctggtg 4980

ggagagactg cagggctgcc agcgtatcgt agaggactgg cagaaaatcc ttatggtgcg 5040

gtcccttgtg gtcagccctc atgaagacat gagaacctgg ctcaagtatg caagcctgtg 5100

cggcaagagt ggcaggctgg ctcttgctca taaaacttta gtgttgctcc tgggagttga 5160

tccgtctcgg caacttgacc atcctctgcc aacagttcac cctcaggtga cctatgccta 5220

catgaaaaac atgtggaaga gtgcccgcaa gatcgatgcc ttccagcaca tgcagcattt 5280

tgtccagacc atgcagcaac aggcccagca tgccatcgct actgaggacc agcagcataa 5340

gcaggaactg cacaagctca tggcccgatg cttcctgaaa cttggagagt ggcagctgaa 5400

tctacagggc atcaatgaga gcacaatccc caaagtgctg cagtactaca gcgccgccac 5460

agagcacgac cgcagctggt acaaggcctg gcatgcgtgg gcagtgatga acttcgaagc 5520

tgtgctacac tacaaacatc agaaccaagc ccgcgatgag aagaagaaac tgcgtcatgc 5580

cagcggggcc aacatcacca acgccaccac tgccgccacc acggccgcca ctgccaccac 5640

cactgccagc accgagggca gcaacagtga gagcgaggcc gagagcaccg agaacagccc 5700

caccccatcg ccgctgcaga agaaggtcac tgaggatctg tccaaaaccc tcctgatgta 5760

cacggtgcct gccgtccagg gcttcttccg ttccatctcc ttgtcacgag gcaacaacct 5820

ccaggataca ctcagagttc tcaccttatg gtttgattat ggtcactggc cagatgtcaa 5880

tgaggcctta gtggaggggg tgaaagccat ccagattgat acctggctac aggttatacc 5940

tcagctcatt gcaagaattg atacgcccag acccttggtg ggacgtctca ttcaccagct 6000

tctcacagac attggtcggt accaccccca ggccctcatc tacccactga cagtggcttc 6060

taagtctacc acgacagccc ggcacaatgc agccaacaag attctgaaga acatgtgtga 6120

gcacagcaac accctggtcc agcaggccat gatggtgagc gaggagctga tccgagtggc 6180

catcctctgg catgagatgt ggcatgaagg cctggaagag gcatctcgtt tgtactttgg 6240

ggaaaggaac gtgaaaggca tgtttgaggt gctggagccc ttgcatgcta tgatggaacg 6300

gggcccccag actctgaagg aaacatcctt taatcaggcc tatggtcgag atttaatgga 6360

ggcccaagag tggtgcagga agtacatgaa atcagggaat gtcaaggacc tcacccaagc 6420

ctgggacctc tattatcatg tgttccgacg aatctcaaag cagctgcctc agctcacatc 6480

cttagagctg caatatgttt ccccaaaact tctgatgtgc cgggaccttg aattggctgt 6540

gccaggaaca tatgacccca accagccaat cattcgcatt cagtccatag caccgtcttt 6600

gcaagtcatc acatccaagc agaggccccg gaaattgaca cttatgggca gcaacggaca 6660

tgagtttgtt ttccttctaa aaggccatga agatctgcgc caggatgagc gtgtgatgca 6720

gctcttcggc ctggttaaca cccttctggc caatgaccca acatctcttc ggaaaaacct 6780

cagcatccag agatacgctg tcatcccttt atcgaccaac tcgggcctca ttggctgggt 6840
tccccactgt gacacactgc acgccctcat ccgggactac agggagaaga agaagatcct 6900
tctcaacatc gagcatcgca tcatgttgcg gatggctccg gactatgacc acttgactct 6960
gatgcagaag gtggaggtgt ttgagcatgc cgtcaataat acagctgggg acgacctggc 7020
caagctgctg tggctgaaaa gccccagctc cgaggtgtgg tttgaccgaa gaaccaatta 7080
tacccgttct ttagcggtca tgtcaatggt tgggtatatt ttaggcctgg gagatagaca 7140
cccatccaac ctgatgctgg accgtctgag tgggaagatc ctgcacattg actttgggga 7200
ctgctttgag gttgctatga cccgagagaa gtttccagag aagattccat ttagactaac 7260
aagaatgttg accaatgcta tggaggttac aggcctggat ggcaactaca gaatcacatg 7320
ccacacagtg atggaggtgc tgcgagagca caaggacagt gtcatggccg tgctggaagc 7380
ctttgtctat gaccccttgc tgaactggag gctgatggac acaaatacca aaggcaacaa 7440
gcgatcccga acgaggacgg attcctactc tgctggccag tcagtcgaaa ttttggacgg 7500
tgtggaactt ggagagccag cccataagaa aacggggacc acagtgccag aatctattca 7560
ttctttcatt ggagacggtt tggtgaaacc agaggcccta aataagaaag ctatccagat 7620
tattaacagg gttcgagata agctcactgg tcgggacttc tctcatgatg acactttgga 7680
tgttccaacg caagttgagc tgctcatcaa acaagcgaca tcccatgaaa acctctgcca 7740
gtgctatatt ggctggtgcc ctttctggta actggaggcc cagatgtgcc catcacgttt 7800
tttctgaggc ttttgtactt tagtaaatgc ttccactaaa ctgaaaccat ggtgagaaag 7860
tttgactttg ttaaatattt tgaaatgtaa atgaaaagaa ctactgtata ttaaaagttg 7920
gtttgaacca actttctagc tgctgttgaa gaatatattg tcagaaacac aaggcttgat 7980
ttggttccca ggacagtgaa acatagtaat accacgtaaa tcaagccatt cattttgggg 8040
aacagaagat ccataacttt agaaatacgg gttttgactt aactcacaag agaactcatc 8100
ataagtactt gctgatggaa gaatgaccta gttgctcctc tcaacatggg tacagcaaac 8160
tcagcacagc caagaagcct caggtcgtgg agaacatgga ttaggatcct agactgtaaa 8220
gacacagaag atgctgacct cacccctgcc acctatccca agacctcact ggtctgtgga 8280
cagcagcaga aatgtttgca agataggcca aaatgagtac aaaaggtctg tcttccatca 8340
gacccagtga tgctgcgact cacacgcttc aattcaagac ctgaccgcta gtagggaggt 8400
ttattcagat cgctggcagc ctcggctgag cagatgcaca gaggggatca ctgtgcagtg 8460
ggaccaccct cactggcctt ctgcagcagg gttctgggat gttttcagtg gtcaaaatac 8520
tctgtttaga gcaagggctc agaaaacaga aatactgtca tggaggtgct gaacacaggg 8580
aaggtctggt acatattgga aattatgagc agaacaaata ctcaactaaa tgcacaaagt 8640

ataaagtgta gccatgtcta gacaccatgt tgtatcagaa taatttttgt gccaataaat 8700

gacatcagaa ttttaaacat atgtaaaaaa aaa 8733

<210> 2
<211> 6055
<212> RNA
<213> Homo sapiens

<220>
<221> mRNA
<222> (1)..(6055)
<223> kinase insert domain receptor (a type III receptor tyrosine
kinase) (VEGFR-2)

<400> 2
actgagtccc gggaccccgg gagagcggtc aatgtgtggt cgctgcgttt cctctgcctg 60

cgccgggcat cacttgcgcg ccgcagaaag tccgtctggc agcctggata tcctctccta 120

ccggcacccg cagacgcccc tgcagccgcg gtcggcgccc gggctcccta gccctgtgcg 180

ctcaactgtc ctgcgctgcg gggtgccgcg agttccacct ccgcgcctcc ttctctagac 240

aggcgctggg agaaagaacc ggctcccgag ttctgggcat ttcgcccggc tcgaggtgca 300

ggatgcagag caaggtgctg ctggccgtcg ccctgtggct ctgcgtggag acccgggccg 360

cctctgtggg tttgcctagt gtttctcttg atctgcccag gctcagcata caaaaagaca 420

tacttacaat taaggctaat acaactcttc aaattacttg caggggacag agggacttgg 480

actggctttg gcccaataat cagagtggca gtgagcaaag ggtggaggtg actgagtgca 540

gcgatggcct cttctgtaag acactcacaa ttccaaaagt gatcggaaat gacactggag 600

cctacaagtg cttctaccgg gaaactgact tggcctcggt catttatgtc tatgttcaag 660

attacagatc tccatttatt gcttctgtta gtgaccaaca tggagtcgtg tacattactg 720

agaacaaaaa caaaactgtg gtgattccat gtctcgggtc catttcaaat ctcaacgtgt 780

cactttgtgc aagataccca gaaaagagat ttgttcctga tggtaacaga atttcctggg 840

acagcaagaa gggctttact attcccagct acatgatcag ctatgctggc atggtcttct 900

gtgaagcaaa aattaatgat gaaagttacc agtctattat gtacatagtt gtcgttgtag 960

ggtataggat ttatgatgtg gttctgagtc cgtctcatgg aattgaacta tctgttggag 1020

aaaagcttgt cttaaattgt acagcaagaa ctgaactaaa tgtggggatt gacttcaact 1080

gggaataccc ttcttcgaag catcagcata agaaacttgt aaaccgagac ctaaaaaccc 1140

agtctgggag tgagatgaag aaatttttga gcaccttaac tatagatggt gtaacccgga 1200

gtgaccaagg attgtacacc tgtgcagcat ccagtgggct gatgaccaag aagaacagca 1260

catttgtcag ggtccatgaa aaaccttttg ttgcttttgg aagtggcatg gaatctctgg 1320

tggaagccac ggtgggggag cgtgtcagaa tccctgcgaa gtaccttggt tacccacccc 1380

cagaaataaa atggtataaa aatggaatac cccttgagtc caatcacaca attaaagcgg 1440

ggcatgtact gacgattatg gaagtgagtg aaagagacac aggaaattac actgtcatcc 1500

ttaccaatcc catttcaaag gagaagcaga gccatgtggt ctctctggtt gtgtatgtcc 1560

caccccagat tggtgagaaa tctctaatct ctcctgtgga ttcctaccag tacggcacca 1620

ctcaaacgct gacatgtacg gtctatgcca ttcctccccc gcatcacatc cactggtatt 1680

ggcagttgga ggaagagtgc gccaacgagc ccagccaagc tgtctcagtg acaaacccat 1740

acccttgtga agaatggaga agtgtggagg acttccaggg aggaaataaa attgaagtta 1800

ataaaaatca atttgctcta attgaaggaa aaaacaaaac tgtaagtacc cttgttatcc 1860

aagcggcaaa tgtgtcagct ttgtacaaat gtgaagcggt caacaaagtc gggagaggag 1920

agagggtgat ctccttccac gtgaccaggg gtcctgaaat tactttgcaa cctgacatgc 1980

agcccactga gcaggagagc gtgtctttgt ggtgcactgc agacagatct acgtttgaga 2040

acctcacatg gtacaagctt ggcccacagc ctctgccaat ccatgtggga gagttgccca 2100

cacctgtttg caagaacttg gatactcttt ggaaattgaa tgccaccatg ttctctaata 2160

gcacaaatga cattttgatc atggagctta agaatgcatc cttgcaggac caaggagact 2220

atgtctgcct tgctcaagac aggaagacca agaaaagaca ttgcgtggtc aggcagctca 2280

cagtcctaga gcgtgtggca cccacgatca caggaaacct ggagaatcag acgacaagta 2340

ttggggaaag catcgaagtc tcatgcacgg catctgggaa tccccctcca cagatcatgt 2400

ggtttaaaga taatgagacc cttgtagaag actcaggcat tgtattgaag gatgggaacc 2460

ggaacctcac tatccgcaga gtgaggaagg aggacgaagg cctctacacc tgccaggcat 2520

gcagtgttct tggctgtgca aaagtggagg catttttcat aatagaaggt gcccaggaaa 2580

agacgaactt ggaaatcatt attctagtag gcacggcggt gattgccatg ttcttctggc 2640

tacttcttgt catcatccta cggaccgtta agcgggccaa tggaggggaa ctgaagacag 2700

gctacttgtc catcgtcatg gatccagatg aactcccatt ggatgaacat tgtgaacgac 2760

tgccttatga tgccagcaaa tgggaattcc ccagagaccg gctgaagcta ggtaagcctc 2820

ttggccgtgg tgcctttggc caagtgattg aagcagatgc ctttggaatt gacaagacag 2880

caacttgcag gacagtagca gtcaaaatgt tgaaagaagg agcaacacac agtgagcatc 2940

gagctctcat gtctgaactc aagatcctca ttcatattgg tcaccatctc aatgtggtca 3000

accttctagg tgcctgtacc aagccaggag ggccactcat ggtgattgtg gaattctgca 3060

aatttggaaa cctgtccact tacctgagga gcaagagaaa tgaatttgtc ccctacaaga 3120

ccaaaggggc acgattccgt caagggaaag actacgttgg agcaatccct gtggatctga 3180

aacggcgctt ggacagcatc accagtagcc agagctcagc cagctctgga tttgtggagg 3240

agaagtccct cagtgatgta gaagaagagg aagctcctga agatctgtat aaggacttcc 3300

tgaccttgga gcatctcatc tgttacagct tccaagtggc taagggcatg gagttcttgg 3360

catcgcgaaa gtgtatccac agggacctgg cggcacgaaa tatcctctta tcggagaaga 3420

acgtggttaa aatctgtgac tttggcttgg cccgggatat ttataaagat ccagattatg 3480

tcagaaaagg agatgctcgc ctccctttga aatggatggc cccagaaaca atttttgaca 3540

gagtgtacac aatccagagt gacgtctggt cttttggtgt tttgctgtgg gaaatatttt 3600

ccttaggtgc ttctccatat cctggggtaa agattgatga agaattttgt aggcgattga 3660

aagaaggaac tagaatgagg gcccctgatt atactacacc agaaatgtac cagaccatgc 3720

tggactgctg gcacggggag cccagtcaga gacccacgtt ttcagagttg gtggaacatt 3780

tgggaaatct cttgcaagct aatgctcagc aggatggcaa agactacatt gttcttccga 3840

tatcagagac tttgagcatg gaagaggatt ctggactctc tctgcctacc tcacctgttt 3900

cctgtatgga ggaggaggaa gtatgtgacc ccaaattcca ttatgacaac acagcaggaa 3960

tcagtcagta tctgcagaac agtaagcgaa agagccggcc tgtgagtgta aaaacatttg 4020

aagatatccc gttagaagaa ccagaagtaa aagtaatccc agatgacaac cagacggaca 4080

gtggtatggt tcttgcctca gaagagctga aaactttgga agacagaacc aaattatctc 4140

catcttttgg tggaatggtg cccagcaaaa gcagggagtc tgtggcatct gaaggctcaa 4200

accagacaag cggctaccag tccggatatc actccgatga cacagacacc accgtgtact 4260

ccagtgagga agcagaactt ttaaagctga tagagattgg agtgcaaacc ggtagcacag 4320

cccagattct ccagcctgac tcggggacca cactgagctc tcctcctgtt taaaaggaag 4380

catccacacc cccaactcct ggacatcaca tgagaggtgc tgctcagatt ttcaagtgtt 4440

gttctttcca ccagcaggaa gtagccgcat ttgattttca tttcgacaac agaaaaagga 4500

cctcggactg cagggagcca gtcttctagg catatcctgg aagaggcttg tgacccaaga 4560

atgtgtctgt gtcttctccc agtgttgacc tgatcctctt tttcattcat ttaaaaagca 4620

tttatcatgc cccctgctgc gggtctcacc atgggtttag aacaaagacg ttcaagaaat 4680

ggccccatcc tcaaagaagt agcagtacct ggggagctga cacttctgta aaactagaag 4740

ataaaccagg caatgtaagt gttcgaggtg ttgaagatgg gaaggatttg cagggctgag 4800

tctatccaag aggctttgtt taggacgtgg gtcccaagcc aagccttaag tgtggaattc 4860

ggattgatag aaaggaagac taacgttacc ttgctttgga gagtactgga gcctgcaaat 4920

gcattgtgtt tgctctggtg gaggtgggca tggggtctgt tctgaaatgt aaagggttca 4980

gacggggttt ctggttttag aaggttgcgt gttcttcgag ttgggctaaa gtagagttcg 5040

ttgtgctgtt tctgactcct aatgagagtt ccttccagac cgttacgtgt ctcctggcca 5100

agccccagga aggaaatgat gcagctctgg ctccttgtct cccaggctga tcctttattc 5160

agaataccac aaagaaagga cattcagctc aaggctccct gccgtgttga agagttctga 5220

ctgcacaaac cagcttctgg tttcttctgg aatgaatacc ctcatatctg tcctgatgtg 5280

atatgtctga gactgaatgc gggaggttca atgtgaagct gtgtgtggtg tcaaagtttc 5340

aggaaggatt ttaccctttt gttcttcccc ctgtccccaa cccactctca ccccgcaacc 5400

catcagtatt ttagttattt ggcctctact ccagtaaacc tgattgggtt tgttcactct 5460

ctgaatgatt attagccaga cttcaaaatt attttatagc ccaaattata acatctattg 5520

tattatttag acttttaaca tatagagcta tttctactga tttttgccct tgttctgtcc 5580

tttttttcaa aaaagaaaat gtgttttttg tttggtacca tagtgtgaaa tgctgggaac 5640

aatgactata agacatgcta tggcacatat atttatagtc tgtttatgta gaaacaaatg 5700

taatatatta aagccttata tataatgaac tttgtactat tcacattttg tatcagtatt 5760

atgtagcata acaaaggtca taatgctttc agcaattgat gtcattttat taaagaacat 5820

tgaaaaactt gaaggaatcc ctttgcaagg ttgcattact gtacccatca tttctaaaat 5880

ggaagagggg gtggctgggc acagtggccg acacctaaaa acccagcact ttggggggcc 5940

aaggtgggag gatcgcttga gcccaggagt tcaagaccag tctggccaac atggtcagat 6000

tccatctcaa agaaaaaagg taaaaataaa ataaaatgga gaagaaggaa tcaga 6055

<210> 3
<211> 5718
<212> RNA
<213> Homo sapiens

<220>
<221> mRNA
<222> (1)..(5718)
<223> platelet-derived growth factor receptor, beta polypeptide
(PDGFRB)

<400> 3
ctcctgaggc tgccagcagc cagcagtgac tgcccgccct atctgggacc caggatcgct 60

ctgtgagcaa cttggagcca gagaggagat caacaaggag gaggagagag ccggcccctc 120

agccctgctg cccagcagca gcctgtgctc gccctgccca acgcagacag ccagacccag 180

ggcggcccct ctggcggctc tgctcctccc gaaggatgct tggggagtga ggcgaagctg 240

ggccgctcct ctcccctaca gcagccccct tcctccatcc ctctgttctc ctgagccttc 300

aggagcctgc accagtcctg cctgtccttc tactcagctg ttacccactc tgggaccagc 360

agtctttctg ataactggga gagggcagta aggaggactt cctggagggg gtgactgtcc 420

agagcctgga actgtgccca caccagaagc catcagcagc aaggacacca tgcggcttcc 480

gggtgcgatg ccagctctgg ccctcaaagg cgagctgctg ttgctgtctc tcctgttact 540

tctggaacca cagatctctc agggcctggt cgtcacaccc ccggggccag agcttgtcct 600
caatgtctcc agcaccttcg ttctgacctg ctcgggttca gctccggtgg tgtgggaacg 660
gatgtcccag gagcccccac aggaaatggc caaggcccag gatggcacct tctccagcgt 720
gctcacactg accaacctca ctgggctaga cacgggagaa tacttttgca cccacaatga 780
ctcccgtgga ctggagaccg atgagcggaa acggctctac atctttgtgc cagatcccac 840
cgtgggcttc ctccctaatg atgccgagga actattcatc tttctcacgg aaataactga 900
gatcaccatt ccatgccgag taacagaccc acagctggtg gtgacactgc acgagaagaa 960
aggggacgtt gcactgcctg tcccctatga tcaccaacgt ggcttttctg gtatctttga 1020
ggacagaagc tacatctgca aaaccaccat tggggacagg gaggtggatt ctgatgccta 1080
ctatgtctac agactccagg tgtcatccat caacgtctct gtgaacgcag tgcagactgt 1140
ggtccgccag ggtgagaaca tcaccctcat gtgcattgtg atcgggaatg aggtggtcaa 1200
cttcgagtgg acataccccc gcaaagaaag tgggcggctg gtggagccgg tgactgactt 1260
cctcttggat atgccttacc acatccgctc catcctgcac atccccagtg ccgagttaga 1320
agactcgggg acctacacct gcaatgtgac ggagagtgtg aatgaccatc aggatgaaaa 1380
ggccatcaac atcaccgtgg ttgagagcgg ctacgtgcgg ctcctgggag aggtgggcac 1440
actacaattt gctgagctgc atcggagccg gacactgcag gtagtgttcg aggcctaccc 1500
accgcccact gtcctgtggt tcaaagacaa ccgcaccctg ggcgactcca gcgctggcga 1560
aatcgccctg tccacgcgca acgtgtcgga gacccggtat gtgtcagagc tgacactggt 1620
tcgcgtgaag gtggcagagg ctggccacta caccatgcgg gccttccatg aggatgctga 1680
ggtccagctc tccttccagc tacagatcaa tgtccctgtc cgagtgctgg agctaagtga 1740
gagccaccct gacagtgggg aacagacagt ccgctgtcgt ggccggggca tgccccagcc 1800
gaacatcatc tggtctgcct gcagagacct caaaaggtgt ccacgtgagc tgccgcccac 1860
gctgctgggg aacagttccg aagaggagag ccagctggag actaacgtga cgtactggga 1920
ggaggagcag gagtttgagg tggtgagcac actgcgtctg cagcacgtgg atcggccact 1980
gtcggtgcgc tgcacgctgc gcaacgctgt gggccaggac acgcaggagg tcatcgtggt 2040
gccacactcc ttgcccttta aggtggtggt gatctcagcc atcctggccc tggtggtgct 2100
caccatcatc tcccttatca tcctcatcat gctttggcag aagaagccac gttacgagat 2160
ccgatggaag gtgattgagt ctgtgagctc tgacggccat gagtacatct acgtggaccc 2220
catgcagctg ccctatgact ccacgtggga gctgccgcgg gaccagcttg tgctgggacg 2280
caccctcggc tctggggcct ttgggcaggt ggtggaggcc acggctcatg gcctgagcca 2340
ttctcaggcc acgatgaaag tggccgtcaa gatgcttaaa tccacagccc gcagcagtga 2400
gaagcaagcc cttatgtcgg agctgaagat catgagtcac cttgggcccc acctgaacgt 2460

ggtcaacctg ttgggggcct gcaccaaagg aggacccatc tatatcatca ctgagtactg 2520
ccgctacgga gacctggtgg actacctgca ccgcaacaaa cacaccttcc tgcagcacca 2580
ctccgacaag cgccgcccgc ccagcgcgga gctctacagc aatgctctgc ccgttgggct 2640
ccccctgccc agccatgtgt ccttgaccgg ggagagcgac ggtggctaca tggacatgag 2700
caaggacgag tcggtggact atgtgcccat gctggacatg aaaggagacg tcaaatatgc 2760
agacatcgag tcctccaact acatggcccc ttacgataac tacgttccct ctgcccctga 2820
gaggacctgc cgagcaactt tgatcaacga gtctccagtg ctaagctaca tggacctcgt 2880
gggcttcagc taccaggtgg ccaatggcat ggagtttctg gcctccaaga actgcgtcca 2940
cagagacctg gcggctagga acgtgctcat ctgtgaaggc aagctggtca agatctgtga 3000
ctttggcctg gctcgagaca tcatgcggga ctcgaattac atctccaaag gcagcacctt 3060
tttgccttta aagtggatgg ctccggagag catcttcaac agcctctaca ccaccctgag 3120
cgacgtgtgg tccttcggga tcctgctctg ggagatcttc accttgggtg gcacccctta 3180
cccagagctg cccatgaacg agcagttcta caatgccatc aaacggggtt accgcatggc 3240
ccagcctgcc catgcctccg acgagatcta tgagatcatg cagaagtgct gggaagagaa 3300
gtttgagatt cggcccccct tctcccagct ggtgctgctt ctcgagagac tgttgggcga 3360
aggttacaaa aagaagtacc agcaggtgga tgaggagttt ctgaggagtg accacccagc 3420
catccttcgg tcccaggccc gcttgcctgg gttccatggc ctccgatctc ccctggacac 3480
cagctccgtc ctctatactg ccgtgcagcc caatgagggt gacaacgact atatcatccc 3540
cctgcctgac cccaaacccg aggttgctga cgagggccca ctggagggtt cccccagcct 3600
agccagctcc accctgaatg aagtcaacac ctcctcaacc atctcctgtg acagccccct 3660
ggagccccag gacgaaccag agccagagcc ccagcttgag ctccaggtgg agccggagcc 3720
agagctggaa cagttgccgg attcggggtg ccctgcgcct cgggcggaag cagaggatag 3780
cttcctgtag ggggctggcc cctaccctgc cctgcctgaa gctccccccc tgccagcacc 3840
cagcatctcc tggcctggcc tgaccgggct tcctgtcagc caggctgccc ttatcagctg 3900
tccccttctg gaagctttct gctcctgacg tgttgtgccc caaaccctgg ggctggctta 3960
ggaggcaaga aaactgcagg ggccgtgacc agccctctgc ctccagggag gccaactgac 4020
tctgagccag ggttccccca gggaactcag ttttcccata tgtaagatgg gaaagttagg 4080
cttgatgacc cagaatctag gattctctcc ctggctgaca ggtggggaga ccgaatccct 4140
ccctgggaag attcttggag ttactgaggt ggtaaattaa cttttttctg ttcagccagc 4200
tacccctcaa ggaatcatag ctctctcctc gcacttttat ccacccagga gctagggaag 4260
agaccctagc ctccctggct gctggctgag ctagggccta gccttgagca gtgttgcctc 4320

atccagaaga aagccagtct cctccctatg atgccagtcc ctgcgttccc tggcccgagc 4380

tggtctgggg ccattaggca gcctaattaa tgctggaggc tgagccaagt acaggacacc 4440

cccagcctgc agcccttgcc cagggcactt ggagcacacg cagccatagc aagtgcctgt 4500

gtccctgtcc ttcaggccca tcagtcctgg ggctttttct ttatcaccct cagtcttaat 4560

ccatccacca gagtctagaa ggccagacgg gccccgcatc tgtgatgaga atgtaaatgt 4620

gccagtgtgg agtggccacg tgtgtgtgcc agtatatggc cctggctctg cattggacct 4680

gctatgaggc tttggaggaa tccctcaccc tctctgggcc tcagtttccc cttcaaaaaa 4740

tgaataagtc ggacttatta actctgagtg ccttgccagc actaacattc tagagtattc 4800

caggtggttg cacatttgtc cagatgaagc aaggccatat accctaaact tccatcctgg 4860

gggtcagctg ggctcctggg agattccaga tcacacatca cactctgggg actcaggaac 4920

catgcccctt ccccaggccc ccagcaagtc tcaagaacac agctgcacag gccttgactt 4980

agagtgacag ccggtgtcct ggaaagcccc cagcagctgc cccagggaca tgggaagacc 5040

acgggacctc tttcactacc cacgatgacc tccgggggta tcctgggcaa aagggacaaa 5100

gagggcaaat gagatcacct cctgcagccc accactccag cacctgtgcc gaggtctgcg 5160

tcgaagacag aatggacagt gaggacagtt atgtcttgta aaagacaaga agcttcagat 5220

gggtacccca agaaggatgt gagaggtggg cgctttggag gtttgcccct cacccaccag 5280

ctgccccatc cctgaggcag cgctccatgg gggtatggtt ttgtcactgc ccagacctag 5340

cagtgacatc tcattgtccc cagcccagtg ggcattggag gtgccagggg agtcagggtt 5400

gtagccaaga cgcccccgca cggggagggt tgggaagggg gtgcaggaag ctcaacccct 5460

ctgggcacca accctgcatt gcaggttggc accttacttc cctgggatcc ccagagttgg 5520

tccaaggagg gagagtgggt tctcaatacg gtaccaaaga tataatcacc taggtttaca 5580

aatattttta ggactcacgt taactcacat ttatacagca gaaatgctat tttgtatgct 5640

gttaagtttt tctatctgtg tacttttttt taagggaaag attttaatat taaacctggt 5700

gcttctcact cacaaaaa 5718

<210> 4
<211> 5917
<212> RNA
<213> Homo sapiens

<220>
<221> mRNA
<222> (1)..(5917)
<223> fibroblast growth factor receptor 1 (FGFR1)

<400> 4
agatgcaggg gcgcaaacgc caaaggagac caggctgtag gaagagaagg gcagagcgcc 60

ggacagctcg gcccgctccc cgtcctttgg ggccgcggct ggggaactac aaggcccagc 120

aggcagctgc agggggcgga ggcggaggag ggaccagcgc gggtgggagt gagagagcga 180

gccctcgcgc cccgccggcg catagcgctc ggagcgctct tgcggccaca ggcgcggcgt 240

cctcggcggc gggcggcagc tagcgggagc cgggacgccg gtgcagccgc agcgcgcgga 300

ggaacccggg tgtgccggga gctgggcggc cacgtccgga cgggaccgag acccctcgta 360

gcgcattgcg gcgacctcgc cttccccggc cgcgagcgcg ccgctgcttg aaaagccgcg 420

gaacccaagg acttttctcc ggtccgagct cggggcgccc cgcagggcgc acggtacccg 480

tgctgcagtc gggcacgccg cggcgccggg gcctccgcag ggcgatggag cccggtctgc 540

aaggaaagtg aggcgccgcc gctgcgttct ggaggagggg ggcacaaggt ctggagaccc 600

cgggtggcgg acgggagccc tccccccgcc ccgcctccgg ggcaccagct ccggctccat 660

tgttcccgcc cgggctggag gcgccgagca ccgagcgccg ccgggagtcg agcgccggcc 720

gcggagctct tgcgaccccg ccaggacccg aacagagccc gggggcggcg ggccggagcc 780

ggggacgcgg gcacacgccc gctcgcacaa gccacggcgg actctcccga ggcggaacct 840

ccacgccgag cgagggtcag tttgaaaagg aggatcgagc tcactgtgga gtatccatgg 900

agatgtggag ccttgtcacc aacctctaac tgcagaactg ggatgtggag ctggaagtgc 960

ctcctcttct gggctgtgct ggtcacagcc acactctgca ccgctaggcc gtccccgacc 1020

ttgcctgaac aagcccagcc ctggggagcc cctgtggaag tggagtcctt cctggtccac 1080

cccggtgacc tgctgcagct tcgctgtcgg ctgcgggacg atgtgcagag catcaactgg 1140

ctgcgggacg gggtgcagct ggcggaaagc aaccgcaccc gcatcacagg ggaggaggtg 1200

gaggtgcagg actccgtgcc cgcagactcc ggcctctatg cttgcgtaac cagcagcccc 1260

tcgggcagtg acaccaccta cttctccgtc aatgtttcag atgctctccc ctcctcggag 1320

gatgatgatg atgatgatga ctcctcttca gaggagaaag aaacagataa caccaaacca 1380

aaccgtatgc ccgtagctcc atattggaca tccccagaaa agatggaaaa gaaattgcat 1440

gcagtgccgg ctgccaagac agtgaagttc aaatgccctt ccagtgggac cccaaacccc 1500

acactgcgct ggttgaaaaa tggcaaagaa ttcaaacctg accacagaat tggaggctac 1560

aaggtccgtt atgccacctg gagcatcata atggactctg tggtgccctc tgacaagggc 1620

aactacacct gcattgtgga gaatgagtac ggcagcatca accacacata ccagctggat 1680

gtcgtggagc ggtcccctca ccggcccatc ctgcaagcag ggttgcccgc caacaaaaca 1740

gtggccctgg gtagcaacgt ggagttcatg tgtaaggtgt acagtgaccc gcagccgcac 1800

atccagtggc taaagcacat cgaggtgaat gggagcaaga ttggcccaga caacctgcct 1860

tatgtccaga tcttgaagac tgctggagtt aataccaccg acaaagagat ggaggtgctt 1920

cacttaagaa atgtctcctt tgaggacgca ggggagtata cgtgcttggc gggtaactct 1980

atcggactct cccatcactc tgcatggttg accgttctgg aagccctgga agagaggccg 2040

gcagtgatga cctcgcccct gtacctggag atcatcatct attgcacagg ggccttcctc 2100

atctcctgca tggtggggtc ggtcatcgtc tacaagatga agagtggtac caagaagagt 2160

gacttccaca gccagatggc tgtgcacaag ctggccaaga gcatccctct gcgcagacag 2220

gtaacagtgt ctgctgactc cagtgcatcc atgaactctg gggttcttct ggttcggcca 2280

tcacggctct cctccagtgg gactcccatg ctagcagggg tctctgagta tgagcttccc 2340

gaagaccctc gctgggagct gcctcgggac agactggtct taggcaaacc cctgggagag 2400

ggctgctttg ggcaggtggt gttggcagag gctatcgggc tggacaagga caaacccaac 2460

cgtgtgacca aagtggctgt gaagatgttg aagtcggacg caacagagaa agacttgtca 2520

gacctgatct cagaaatgga gatgatgaag atgatcggga agcataagaa tatcatcaac 2580

ctgctggggg cctgcacgca ggatggtccc ttgtatgtca tcgtggagta tgcctccaag 2640

ggcaacctgc gggagtacct gcaggcccgg aggcccccag ggctggaata ctgctacaac 2700

cccagccaca acccagagga gcagctctcc tccaaggacc tggtgtcctg cgcctaccag 2760

gtggcccgag gcatggagta tctggcctcc aagaagtgca tacaccgaga cctggcagcc 2820

aggaatgtcc tggtgacaga ggacaatgtg atgaagatag cagactttgg cctcgcacgg 2880

gacattcacc acatcgacta ctataaaaag acaaccaacg gccgactgcc tgtgaagtgg 2940

atggcacccg aggcattatt tgaccggatc tacacccacc agagtgatgt gtggtctttc 3000

ggggtgctcc tgtgggagat cttcactctg ggcggctccc cataccccgg tgtgcctgtg 3060

gaggaacttt tcaagctgct gaaggagggt caccgcatgg acaagcccag taactgcacc 3120

aacgagctgt acatgatgat gcgggactgc tggcatgcag tgccctcaca gagacccacc 3180

ttcaagcagc tggtggaaga cctggaccgc atcgtggcct tgacctccaa ccaggagtac 3240

ctggacctgt ccatgcccct ggaccagtac tcccccagct ttcccgacac ccggagctct 3300

acgtgctcct caggggagga ttccgtcttc tctcatgagc cgctgcccga ggagccctgc 3360

ctgccccgac acccagccca gcttgccaat ggcggactca aacgccgctg actgccaccc 3420

acacgccctc cccagactcc accgtcagct gtaaccctca cccacagccc ctgctgggcc 3480

caccacctgt ccgtccctgt cccctttcct gctggcagga gccggctgcc taccaggggc 3540

cttcctgtgt ggcctgcctt caccccactc agctcacctc tccctccacc tcctctccac 3600

ctgctggtga gaggtgcaaa gaggcagatc tttgctgcca gccacttcat cccctcccag 3660

atgttggacc aacacccctc cctgccacca ggcactgcct ggagggcagg gagtgggagc 3720

caatgaacag gcatgcaagt gagagcttcc tgagctttct cctgtcggtt tggtctgttt 3780

tgccttcacc cataagcccc tcgcactctg gtggcaggtg ccttgtcctc agggctacag 3840

cagtagggag gtcagtgctt cgtgcctcga ttgaaggtga cctctgcccc agataggtgg 3900

tgccagtggc ttattaattc cgatactagt ttgctttgct gaccaaatgc ctggtaccag 3960

aggatggtga ggcgaaggcc aggttggggg cagtgttgtg gccctggggc ccagccccaa 4020

actgggggct ctgtatatag ctatgaagaa aacacaaagt gtataaatct gagtatatat 4080

ttacatgtct ttttaaaagg gtcgttacca gagatttacc catcgggtaa gatgctcctg 4140

gtggctggga ggcatcagtt gctatatatt aaaaacaaaa aagaaaaaaa aggaaaatgt 4200

ttttaaaaag gtcatatatt ttttgctact tttgctgttt tattttttta aattatgttc 4260

taaacctatt ttcagtttag gtccctcaat aaaaattgct gctgcttcat ttatctatgg 4320

gctgtatgaa aagggtggga atgtccactg gaaagaaggg acacccacgg gccctggggc 4380

taggtctgtc ccgagggcac cgcatgctcc cggcgcaggt tccttgtaac ctcttcttcc 4440

taggtcctgc acccagacct cacgacgcac ctcctgcctc tccgctgctt ttggaaagtc 4500

agaaaaagaa gatgtctgct tcgagggcag gaaccccatc catgcagtag aggcgctggg 4560

cagagagtca aggcccagca gccatcgacc atggatggtt tcctccaagg aaaccggtgg 4620

ggttgggctg gggagggggc acctacctag gaatagccac ggggtagagc tacagtgatt 4680

aagaggaaag caagggcgcg gttgctcacg cctgtaatcc cagcactttg ggacaccgag 4740

gtgggcagat cacttcaggt caggagtttg agaccagcct ggccaactta gtgaaacccc 4800

atctctacta aaaatgcaaa aattatccag gcatggtggc acacgcctgt aatcccagct 4860

ccacaggagg ctgaggcaga atcccttgaa gctgggaggc ggaggttgca gtgagccgag 4920

attgcgccat tgcactccag cctgggcaac agagaaaaca aaaaggaaaa caaatgatga 4980

aggtctgcag aaactgaaac ccagacatgt gtctgccccc tctatgtggg catggttttg 5040

ccagtgcttc taagtgcagg agaacatgtc acctgaggct agttttgcat tcaggtccct 5100

ggcttcgttt cttgttggta tgcctcccca gatcgtcctt cctgtatcca tgtgaccaga 5160

ctgtatttgt tgggactgtc gcagatcttg gcttcttaca gttcttcctg tccaaactcc 5220

atcctgtccc tcaggaacgg ggggaaaatt ctccgaatgt ttttggtttt ttggctgctt 5280

ggaatttact tctgccacct gctggtcatc actgtcctca ctaagtggat tctggctccc 5340

ccgtacctca tggctcaaac taccactcct cagtcgctat attaaagctt atattttgct 5400

ggattactgc taaatacaaa agaaagttca atatgttttc atttctgtag ggaaaatggg 5460

attgctgctt taaatttctg agctagggat tttttggcag ctgcagtgtt ggcgactatt 5520

gtaaaattct ctttgtttct ctctgtaaat agcacctgct aacattacaa tttgtattta 5580

tgtttaaaga aggcatcatt tggtgaacag aactaggaaa tgaattttta gctcttaaaa 5640

gcatttgctt tgagaccgca caggagtgtc tttccttgta aaacagtgat gataatttct 5700

gccttggccc taccttgaag caatgttgtg tgaagggatg aagaatctaa aagtcttcat 5760

aagtccttgg gagaggtgct agaaaaatat aaggcactat cataattaca gtgatgtcct 5820

tgctgttact actcaaatca cccacaaatt tccccaaaga ctgcgctagc tgtcaaataa 5880

aagacagtga aattgacctg aaaaaaaaaa aaaaaaa 5917

<210> 5
<211> 5190
<212> RNA
<213> Homo sapiens

<220>
<221> mRNA
<222> (1)..(5190)
<223> v-kit Hardy-Zuckerman 4 feline sarcoma viral oncogene homolog
(cKIT)

<400> 5
tctgggggct cggctttgcc gcgctcgctg cacttgggcg agagctggaa cgtggaccag 60

agctcggatc ccatcgcagc taccgcgatg agaggcgctc gcggcgcctg ggattttctc 120

tgcgttctgc tcctactgct tcgcgtccag acaggctctt ctcaaccatc tgtgagtcca 180

ggggaaccgt ctccaccatc catccatcca ggaaaatcag acttaatagt ccgcgtgggc 240

gacgagatta ggctgttatg cactgatccg ggctttgtca aatggacttt tgagatcctg 300

gatgaaacga atgagaataa gcagaatgaa tggatcacgg aaaaggcaga agccaccaac 360

accggcaaat acacgtgcac caacaaacac ggcttaagca attccattta tgtgtttgtt 420

agagatcctg ccaagctttt ccttgttgac cgctccttgt atgggaaaga agacaacgac 480

acgctggtcc gctgtcctct cacagaccca gaagtgacca attattccct caaggggtgc 540

caggggaagc ctcttcccaa ggacttgagg tttattcctg accccaaggc gggcatcatg 600

atcaaaagtg tgaaacgcgc ctaccatcgg ctctgtctgc attgttctgt ggaccaggag 660

ggcaagtcag tgctgtcgga aaaattcatc ctgaaagtga ggccagcctt caaagctgtg 720

cctgttgtgt ctgtgtccaa agcaagctat cttcttaggg aaggggaaga attcacagtg 780

acgtgcacaa taaaagatgt gtctagttct gtgtactcaa cgtggaaaag agaaaacagt 840

cagactaaac tacaggagaa atataatagc tggcatcacg gtgacttcaa ttatgaacgt 900

caggcaacgt tgactatcag ttcagcgaga gttaatgatt ctggagtgtt catgtgttat 960

gccaataata cttttggatc agcaaatgtc acaacaacct tggaagtagt agataaagga 1020

ttcattaata tcttccccat gataaacact acagtatttg taaacgatgg agaaaatgta 1080

gatttgattg ttgaatatga agcattcccc aaacctgaac accagcagtg gatctatatg 1140

aacagaacct tcactgataa atgggaagat tatcccaagt ctgagaatga aagtaatatc 1200

agatacgtaa gtgaacttca tctaacgaga ttaaaaggca ccgaaggagg cacttacaca 1260

ttcctagtgt ccaattctga cgtcaatgct gccatagcat ttaatgttta tgtgaataca 1320

aaaccagaaa tcctgactta cgacaggctc gtgaatggca tgctccaatg tgtggcagca 1380

ggattcccag agcccacaat agattggtat ttttgtccag gaactgagca gagatgctct 1440

gcttctgtac tgccagtgga tgtgcagaca ctaaactcat ctgggccacc gtttggaaag 1500

ctagtggttc agagttctat agattctagt gcattcaagc acaatggcac ggttgaatgt 1560

aaggcttaca acgatgtggg caagacttct gcctatttta actttgcatt taaaggtaac 1620

aacaaagagc aaatccatcc ccacaccctg ttcactcctt tgctgattgg tttcgtaatc 1680

gtagctggca tgatgtgcat tattgtgatg attctgacct acaaatattt acagaaaccc 1740

atgtatgaag tacagtggaa ggttgttgag gagataaatg gaaacaatta tgtttacata 1800

gacccaacac aacttcctta tgatcacaaa tgggagtttc ccagaaacag gctgagtttt 1860

gggaaaaccc tgggtgctgg agctttcggg aaggttgttg aggcaactgc ttatggctta 1920

attaagtcag atgcggccat gactgtcgct gtaaagatgc tcaagccgag tgcccatttg 1980

acagaacggg aagccctcat gtctgaactc aaagtcctga gttaccttgg taatcacatg 2040

aatattgtga atctacttgg agcctgcacc attggagggc ccaccctggt cattacagaa 2100

tattgttgct atggtgatct tttgaatttt ttgagaagaa aacgtgattc atttatttgt 2160

tcaaagcagg aagatcatgc agaagctgca ctttataaga atcttctgca ttcaaaggag 2220

tcttcctgca gcgatagtac taatgagtac atggacatga aacctggagt ttcttatgtt 2280

gtcccaacca aggccgacaa aaggagatct gtgagaatag gctcatacat agaaagagat 2340

gtgactcccg ccatcatgga ggatgacgag ttggccctag acttagaaga cttgctgagc 2400

ttttcttacc aggtggcaaa gggcatggct ttcctcgcct ccaagaattg tattcacaga 2460

gacttggcag ccagaaatat cctccttact catggtcgga tcacaaagat ttgtgatttt 2520

ggtctagcca gagacatcaa gaatgattct aattatgtgg ttaaaggaaa cgctcgacta 2580

cctgtgaagt ggatggcacc tgaaagcatt ttcaactgtg tatacacgtt tgaaagtgac 2640

gtctggtcct atgggatttt tctttgggag ctgttctctt taggaagcag cccctatcct 2700

ggaatgccgg tcgattctaa gttctacaag atgatcaagg aaggcttccg gatgctcagc 2760

cctgaacacg cacctgctga aatgtatgac ataatgaaga cttgctggga tgcagatccc 2820

ctaaaaagac caacattcaa gcaaattgtt cagctaattg agaagcagat ttcagagagc 2880

accaatcata tttactccaa cttagcaaac tgcagcccca accgacagaa gcccgtggta 2940

gaccattctg tgcggatcaa ttctgtcggc agcaccgctt cctcctccca gcctctgctt 3000

gtgcacgacg atgtctgagc agaatcagtg tttgggtcac ccctccagga atgatctctt 3060

cttttggctt ccatgatggt tattttcttt tctttcaact tgcatccaac tccaggatag 3120

tgggcacccc actgcaatcc tgtctttctg agcacacttt agtggccgat gatttttgtc 3180

atcagccacc atcctattgc aaaggttcca actgtatata ttcccaatag caacgtagct 3240

tctaccatga acagaaaaca ttctgatttg gaaaaagaga gggaggtatg gactgggggc 3300

cagagtcctt tccaaggctt ctccaattct gcccaaaaat atggttgata gtttacctga 3360

ataaatggta gtaatcacag ttggccttca gaaccatcca tagtagtatg atgatacaag 3420

attagaagct gaaaacctaa gtcctttatg tggaaaacag aacatcatta gaacaaagga 3480

cagagtatga acacctgggc ttaagaaatc tagtatttca tgctgggaat gagacatagg 3540

ccatgaaaaa aatgatcccc aagtgtgaac aaaagatgct cttctgtgga ccactgcatg 3600

agcttttata ctaccgacct ggtttttaaa tagagtttgc tattagagca ttgaattgga 3660

gagaaggcct ccctagccag cacttgtata tacgcatcta taaattgtcc gtgttcatac 3720

atttgagggg aaaacaccat aaggtttcgt ttctgtatac aaccctggca ttatgtccac 3780

tgtgtataga agtagattaa gagccatata agtttgaagg aaacagttaa taccattttt 3840

taaggaaaca atataaccac aaagcacagt ttgaacaaaa tctcctcttt tagctgatga 3900

acttattctg tagattctgt ggaacaagcc tatcagcttc agaatggcat tgtactcaat 3960

ggatttgatg ctgtttgaca aagttactga ttcactgcat ggctcccaca ggagtgggaa 4020

aacactgcca tcttagtttg gattcttatg tagcaggaaa taaagtatag gtttagcctc 4080

cttcgcaggc atgtcctgga caccgggcca gtatctatat atgtgtatgt acgtttgtat 4140

gtgtgtagac aaatatttgg aggggtattt ttgccctgag tccaagaggg tcctttagta 4200

cctgaaaagt aacttggctt tcattattag tactgctctt gtttcttttc acatagctgt 4260

ctagagtagc ttaccagaag cttccatagt ggtgcagagg aagtggaagg catcagtccc 4320

tatgtatttg cagttcacct gcacttaagg cactctgtta tttagactca tcttactgta 4380

cctgttcctt agaccttcca taatgctact gtctcactga aacatttaaa ttttaccctt 4440

tagactgtag cctggatatt attcttgtag tttacctctt taaaaacaaa acaaaacaaa 4500

acaaaaaact ccccttcctc actgcccaat ataaaaggca aatgtgtaca tggcagagtt 4560

tgtgtgttgt cttgaaagat tcaggtatgt tgcctttatg gtttccccct tctacatttc 4620

ttagactaca tttagagaac tgtggccgtt atctggaagt aaccatttgc actggagttc 4680

tatgctctcg cacctttcca aagttaacag attttggggt tgtgttgtca cccaagagat 4740

tgttgtttgc catactttgt ctgaaaaatt cctttgtgtt tctattgact tcaatgatag 4800

taagaaaagt ggttgttagt tatagatgtc taggtacttc aggggcactt cattgagagt 4860

tttgtcttgg atattcttga aagtttatat ttttataatt ttttcttaca tcagatgttt 4920

ctttgcagtg gcttaatgtt tgaaattatt ttgtggcttt ttttgtaaat attgaaatgt 4980

agcaataatg tcttttgaat attcccaagc ccatgagtcc ttgaaaatat tttttatata 5040

tacagtaact ttatgtgtaa atacataagc ggcgtaagtt taaaggatgt tggtgttcca 5100

cgtgttttat tcctgtatgt tgtccaattg ttgacagttc tgaagaattc taataaaatg 5160

tacatatata aatcaaaaaa aaaaaaaaaa 5190

<210> 6
<211> 5616
<212> RNA
<213> Homo sapiens

<220>
<221> mRNA
<222> (1)..(5616)
<223> epidermal growth factor receptor (EGFR)

<400> 6
ccccggcgca gcgcggccgc agcagcctcc gccccccgca cggtgtgagc gcccgacgcg 60

gccgaggcgg ccggagtccc gagctagccc cggcggccgc cgccgcccag accggacgac 120

aggccacctc gtcggcgtcc gcccgagtcc ccgcctcgcc gccaacgcca caaccaccgc 180

gcacggcccc ctgactccgt ccagtattga tcgggagagc cggagcgagc tcttcgggga 240

gcagcgatgc gaccctccgg gacggccggg gcagcgctcc tggcgctgct ggctgcgctc 300

tgcccggcga gtcgggctct ggaggaaaag aaagtttgcc aaggcacgag taacaagctc 360

acgcagttgg gcacttttga agatcatttt ctcagcctcc agaggatgtt caataactgt 420

gaggtggtcc ttgggaattt ggaaattacc tatgtgcaga ggaattatga tctttccttc 480

ttaaagacca tccaggaggt ggctggttat gtcctcattg ccctcaacac agtggagcga 540

attcctttgg aaaacctgca gatcatcaga ggaaatatgt actacgaaaa ttcctatgcc 600

ttagcagtct tatctaacta tgatgcaaat aaaaccggac tgaaggagct gcccatgaga 660

aatttacagg aaatcctgca tggcgccgtg cggttcagca acaaccctgc cctgtgcaac 720

gtggagagca tccagtggcg ggacatagtc agcagtgact ttctcagcaa catgtcgatg 780

gacttccaga accacctggg cagctgccaa aagtgtgatc caagctgtcc caatgggagc 840

tgctggggtg caggagagga gaactgccag aaactgacca aaatcatctg tgcccagcag 900

tgctccgggc gctgccgtgg caagtccccc agtgactgct gccacaacca gtgtgctgca 960

ggctgcacag gcccccggga gagcgactgc ctggtctgcc gcaaattccg agacgaagcc 1020

acgtgcaagg acacctgccc cccactcatg ctctacaacc ccaccacgta ccagatggat 1080

gtgaaccccg agggcaaata cagctttggt gccacctgcg tgaagaagtg tccccgtaat 1140

tatgtggtga cagatcacgg ctcgtgcgtc cgagcctgtg gggccgacag ctatgagatg 1200

gaggaagacg gcgtccgcaa gtgtaagaag tgcgaagggc cttgccgcaa agtgtgtaac 1260

ggaataggta ttggtgaatt taaagactca ctctccataa atgctacgaa tattaaacac 1320

ttcaaaaact gcacctccat cagtggcgat ctccacatcc tgccggtggc atttaggggt 1380

gactccttca cacatactcc tcctctggat ccacaggaac tggatattct gaaaaccgta 1440

aaggaaatca cagggttttt gctgattcag gcttggcctg aaaacaggac ggacctccat 1500

gcctttgaga acctagaaat catacgcggc aggaccaagc aacatggtca gttttctctt 1560

gcagtcgtca gcctgaacat aacatccttg ggattacgct ccctcaagga gataagtgat 1620

ggagatgtga taatttcagg aaacaaaaat ttgtgctatg caaatacaat aaactggaaa 1680

aaactgtttg ggacctccgg tcagaaaacc aaaattataa gcaacagagg tgaaaacagc 1740

tgcaaggcca caggccaggt ctgccatgcc ttgtgctccc ccgagggctg ctggggcccg 1800

gagcccaggg actgcgtctc ttgccggaat gtcagccgag gcagggaatg cgtggacaag 1860

tgcaaccttc tggagggtga gccaagggag tttgtggaga actctgagtg catacagtgc 1920

cacccagagt gcctgcctca ggccatgaac atcacctgca caggacgggg accagacaac 1980

tgtatccagt gtgcccacta cattgacggc ccccactgcg tcaagacctg cccggcagga 2040

gtcatgggag aaaacaacac cctggtctgg aagtacgcag acgccggcca tgtgtgccac 2100

ctgtgccatc caaactgcac ctacggatgc actgggccag gtcttgaagg ctgtccaacg 2160

aatgggccta agatcccgtc catcgccact gggatggtgg gggccctcct cttgctgctg 2220

gtggtggccc tggggatcgg cctcttcatg cgaaggcgcc acatcgttcg gaagcgcacg 2280

ctgcggaggc tgctgcagga gagggagctt gtggagcctc ttacacccag tggagaagct 2340

cccaaccaag ctctcttgag gatcttgaag gaaactgaat tcaaaaagat caaagtgctg 2400

ggctccggtg cgttcggcac ggtgtataag ggactctgga tcccagaagg tgagaaagtt 2460

aaaattcccg tcgctatcaa ggaattaaga gaagcaacat ctccgaaagc caacaaggaa 2520

atcctcgatg aagcctacgt gatggccagc gtggacaacc cccacgtgtg ccgcctgctg 2580

ggcatctgcc tcacctccac cgtgcagctc atcacgcagc tcatgccctt cggctgcctc 2640

ctggactatg tccgggaaca caaagacaat attggctccc agtacctgct caactggtgt 2700

gtgcagatcg caaagggcat gaactacttg gaggaccgtc gcttggtgca ccgcgacctg 2760

gcagccagga acgtactggt gaaaacaccg cagcatgtca agatcacaga ttttgggctg 2820

gccaaactgc tgggtgcgga agagaaagaa taccatgcag aaggaggcaa agtgcctatc 2880

aagtggatgg cattggaatc aattttacac agaatctata cccaccagag tgatgtctgg 2940

agctacgggg tgaccgtttg ggagttgatg acctttggat ccaagccata tgacggaatc 3000

cctgccagcg agatctcctc catcctggag aaaggagaac gcctccctca gccacccata 3060

tgtaccatcg atgtctacat gatcatggtc aagtgctgga tgatagacgc agatagtcgc 3120

ccaaagttcc gtgagttgat catcgaattc tccaaaatgg cccgagaccc ccagcgctac 3180

cttgtcattc agggggatga aagaatgcat ttgccaagtc ctacagactc caacttctac 3240

cgtgccctga tggatgaaga agacatggac gacgtggtgg atgccgacga gtacctcatc 3300

ccacagcagg gcttcttcag cagcccctcc acgtcacgga ctcccctcct gagctctctg 3360

agtgcaacca gcaacaattc caccgtggct tgcattgata gaaatgggct gcaaagctgt 3420

cccatcaagg aagacagctt cttgcagcga tacagctcag accccacagg cgccttgact 3480

gaggacagca tagacgacac cttcctccca gtgcctgaat acataaacca gtccgttccc 3540

aaaaggcccg ctggctctgt gcagaatcct gtctatcaca atcagcctct gaaccccgcg 3600

cccagcagag acccacacta ccaggacccc cacagcactg cagtgggcaa ccccgagtat 3660

ctcaacactg tccagcccac ctgtgtcaac agcacattcg acagccctgc ccactgggcc 3720

cagaaaggca gccaccaaat tagcctggac aaccctgact accagcagga cttctttccc 3780

aaggaagcca agccaaatgg catctttaag ggctccacag ctgaaaatgc agaataccta 3840

agggtcgcgc cacaaagcag tgaatttatt ggagcatgac cacggaggat agtatgagcc 3900

ctaaaaatcc agactctttc gatacccagg accaagccac agcaggtcct ccatcccaac 3960

agccatgccc gcattagctc ttagacccac agactggttt tgcaacgttt acaccgacta 4020

gccaggaagt acttccacct cgggcacatt ttgggaagtt gcattccttt gtcttcaaac 4080

tgtgaagcat ttacagaaac gcatccagca agaatattgt ccctttgagc agaaatttat 4140

ctttcaaaga ggtatatttg aaaaaaaaaa aaagtatatg tgaggatttt tattgattgg 4200

ggatcttgga gtttttcatt gtcgctattg atttttactt caatgggctc ttccaacaag 4260

gaagaagctt gctggtagca cttgctaccc tgagttcatc caggcccaac tgtgagcaag 4320

gagcacaagc cacaagtctt ccagaggatg cttgattcca gtggttctgc ttcaaggctt 4380

ccactgcaaa acactaaaga tccaagaagg ccttcatggc cccagcaggc cggatcggta 4440

ctgtatcaag tcatggcagg tacagtagga taagccactc tgtcccttcc tgggcaaaga 4500

agaaacggag gggatggaat tcttccttag acttactttt gtaaaaatgt ccccacggta 4560

cttactcccc actgatggac cagtggtttc cagtcatgag cgttagactg acttgtttgt 4620

cttccattcc attgttttga aactcagtat gctgcccctg tcttgctgtc atgaaatcag 4680

caagagagga tgacacatca aataataact cggattccag cccacattgg attcatcagc 4740

atttggacca atagcccaca gctgagaatg tggaatacct aaggatagca ccgcttttgt 4800

tctcgcaaaa acgtatctcc taatttgagg ctcagatgaa atgcatcagg tcctttgggg 4860

catagatcag aagactacaa aaatgaagct gctctgaaat ctcctttagc catcacccca 4920

accccccaaa attagtttgt gttacttatg gaagatagtt ttctcctttt acttcacttc 4980

aaaagctttt tactcaaaga gtatatgttc cctccaggtc agctgccccc aaaccccctc 5040

cttacgcttt gtcacacaaa aagtgtctct gccttgagtc atctattcaa gcacttacag 5100

ctctggccac aacagggcat tttacaggtg cgaatgacag tagcattatg agtagtgtgg 5160

aattcaggta gtaaatatga aactagggtt tgaaattgat aatgctttca caacatttgc 5220

agatgtttta gaaggaaaaa agttccttcc taaaataatt tctctacaat tggaagattg 5280

gaagattcag ctagttagga gcccaccttt tttcctaatc tgtgtgtgcc ctgtaacctg 5340

actggttaac agcagtcctt tgtaaacagt gttttaaact ctcctagtca atatccaccc 5400

catccaattt atcaaggaag aaatggttca gaaaatattt tcagcctaca gttatgttca 5460

gtcacacaca catacaaaat gttccttttg cttttaaagt aatttttgac tcccagatca 5520

gtcagagccc ctacagcatt gttaagaaag tatttgattt ttgtctcaat gaaaataaaa 5580

ctatattcat ttccactcta aaaaaaaaaa aaaaaa 5616

<210> 7
<211> 3291
<212> RNA
<213> Homo sapiens

<220>
<221> mRNA
<222> (1)..(3291)
<223> Raf-1 proto-oncogene, serine/threonine kinase (RAF1, cRAF)

<400> 7
agaatcggag agccggtggc gtcgcaggtc gggaggacga gcaccgagtc gagggctcgc 60

tcgtctgggc cgcccgagag tcttaatcgc gggcgcttgg gccgccatct tagatggcgg 120

gagtaagagg aaaacgattg tgaggcggga acggctttct gctgcctttt ttgggccccg 180

aaaagggtca gctggccggg ctttggggcg cgtgccctga ggcgcggagc gcgtttgcta 240

cgatgcgggg gctgctcggg gctccgtccc ctgggctggg gacgcgccga atgtgaccgc 300

ctcccgctcc ctcacccgcc gcggggagga ggagcgggcg agaagctgcc gccgaacgac 360

aggacgttgg ggcggcctgg ctccctcagg tttaagaatt gtttaagctg catcaatgga 420

gcacatacag ggagcttgga agacgatcag caatggtttt ggattcaaag atgccgtgtt 480

tgatggctcc agctgcatct ctcctacaat agttcagcag tttggctatc agcgccgggc 540

atcagatgat ggcaaactca cagatccttc taagacaagc aacactatcc gtgttttctt 600

gccgaacaag caaagaacag tggtcaatgt gcgaaatgga atgagcttgc atgactgcct 660

tatgaaagca ctcaaggtga ggggcctgca accagagtgc tgtgcagtgt tcagacttct 720

ccacgaacac aaaggtaaaa aagcacgctt agattggaat actgatgctg cgtctttgat 780

tggagaagaa cttcaagtag atttcctgga tcatgttccc ctcacaacac acaactttgc 840

tcggaagacg ttcctgaagc ttgccttctg tgacatctgt cagaaattcc tgctcaatgg 900

atttcgatgt cagacttgtg gctacaaatt tcatgagcac tgtagcacca aagtacctac 960

tatgtgtgtg gactggagta acatcagaca actcttattg tttccaaatt ccactattgg 1020

tgatagtgga gtcccagcac taccttcttt gactatgcgt cgtatgcgag agtctgtttc 1080

caggatgcct gttagttctc agcacagata ttctacacct cacgccttca cctttaacac 1140

ctccagtccc tcatctgaag gttccctctc ccagaggcag aggtcgacat ccacacctaa 1200

tgtccacatg gtcagcacca ccctgcctgt ggacagcagg atgattgagg atgcaattcg 1260

aagtcacagc gaatcagcct caccttcagc cctgtccagt agccccaaca atctgagccc 1320

aacaggctgg tcacagccga aaacccccgt gccagcacaa agagagcggg caccagtatc 1380

tgggacccag gagaaaaaca aaattaggcc tcgtggacag agagattcaa gctattattg 1440

ggaaatagaa gccagtgaag tgatgctgtc cactcggatt gggtcaggct cttttggaac 1500

tgtttataag ggtaaatggc acggagatgt tgcagtaaag atcctaaagg ttgtcgaccc 1560

aaccccagag caattccagg ccttcaggaa tgaggtggct gttctgcgca aaacacggca 1620

tgtgaacatt ctgcttttca tggggtacat gacaaaggac aacctggcaa ttgtgaccca 1680

gtggtgcgag ggcagcagcc tctacaaaca cctgcatgtc caggagacca agtttcagat 1740

gttccagcta attgacattg cccggcagac ggctcaggga atggactatt tgcatgcaaa 1800

gaacatcatc catagagaca tgaaatccaa caatatattt ctccatgaag gcttaacagt 1860

gaaaattgga gattttggtt tggcaacagt aaagtcacgc tggagtggtt ctcagcaggt 1920

tgaacaacct actggctctg tcctctggat ggccccagag gtgatccgaa tgcaggataa 1980

caacccattc agtttccagt cggatgtcta ctcctatggc atcgtattgt atgaactgat 2040

gacgggggag cttccttatt ctcacatcaa caaccgagat cagatcatct tcatggtggg 2100

ccgaggatat gcctccccag atcttagtaa gctatataag aactgcccca aagcaatgaa 2160

gaggctggta gctgactgtg tgaagaaagt aaaggaagag aggcctcttt ttccccagat 2220

cctgtcttcc attgagctgc tccaacactc tctaccgaag atcaaccgga gcgcttccga 2280

gccatccttg catcgggcag cccacactga ggatatcaat gcttgcacgc tgaccacgtc 2340

cccgaggctg cctgtcttct agttgacttt gcacctgtct tcaggctgcc aggggaggag 2400

gagaagccag caggcaccac ttttctgctc cctttctcca gaggcagaac acatgttttc 2460

agagaagctg ctgctaagga ccttctagac tgctcacagg gccttaactt catgttgcct 2520

tcttttctat ccctttgggc cctgggagaa ggaagccatt tgcagtgctg gtgtgtcctg 2580

ctccctcccc acattcccca tgctcaaggc ccagccttct gtagatgcgc aagtggatgt 2640

tgatggtagt acaaaaagca ggggcccagc cccagctgtt ggctacatga gtatttagag 2700

gaagtaaggt agcaggcagt ccagccctga tgtggagaca catgggattt tggaaatcag 2760

cttctggagg aatgcatgtc acaggcggga ctttcttcag agagtggtgc agcgccagac 2820

attttgcaca taaggcacca aacagcccag gactgccgag actctggccg cccgaaggag 2880

cctgctttgg tactatggaa cttttcttag gggacacgtc ctcctttcac agcttctaag 2940

gtgtccagtg cattgggatg gttttccagg caaggcactc ggccaatccg catctcagcc 3000

ctctcaggga gcagtcttcc atcatgctga attttgtctt ccaggagctg cccctatggg 3060

gcggggccgc agggccagcc ttgtttctct aacaaacaaa caaacaaaca gccttgtttc 3120

tctagtcaca tcatgtgtat acaaggaagc caggaataca ggttttcttg atgatttggg 3180

ttttaatttt gtttttattg cacctgacaa aatacagtta tctgatggtc cctcaattat 3240

gttattttaa taaaataaat taaatttagg tgtaaaaaaa aaaaaaaaaa a 3291

<210> 8
<211> 23
<212> DNA
<213> Artificial Sequence

<220>
<223> VEGFR2 forward primer

<400> 8
caccactcaa acgctgacat gta 23

<210> 9
<211> 22
<212> DNA
<213> Artificial Sequence

<220>
<223> VEGFR2 reverse primer

<400> 9
ccaactgcca ataccagtgg at 22

<210> 10
<211> 23
<212> DNA
<213> Artificial Sequence

<220>
<223> VEGFR2 probe

<400> 10
tatgccattc ctcccccgca tca 23

<210> 11
<211> 16
<212> DNA
<213> Artificial Sequence

<220>
<223> PDGFR-B forward primer

<400>    11
agcgctggcg aaatcg                                                        16


<210>    12
<211>    19
<212>    DNA
<213>    Artificial Sequence

<220>
<223>    PDGFR-B reverse primer


<400>    12
ttcacgcgaa ccagtgtca                                                     19


<210>    13
<211>    21
<212>    DNA
<213>    Artificial Sequence

<220>
<223>    PDGFR-B probe


<400>    13
ctgtccacgc gcaacgtgtc g                                                  21


<210>    14
<211>    23
<212>    DNA
<213>    Artificial Sequence

<220>
<223>    C-KIT forward primer


<400>    14
cagatttcag agagcaccaa tca                                                23


<210>    15
<211>    21
<212>    DNA
<213>    Artificial Sequence

<220>
<223>    C-KIT reverse primer


<400>    15
aatggtctac cacgggcttc t                                                  21


<210>    16
<211>    30
<212>    DNA
<213>    Artificial Sequence

<220>

<223>    C-KIT probe

<400>    16
ttactccaac ttagcaaact gcagccccaa                                        30

<210>    17
<211>    23
<212>    DNA
<213>    Artificial Sequence

<220>
<223>    C-RAF forward primer

<400>    17
gaggtcgaca tccacaccta atg                                               23

<210>    18
<211>    22
<212>    DNA
<213>    Artificial Sequence

<220>
<223>    C-RAF reverse primer

<400>    18
tcgaattgca tcctcaatca tc                                                22

<210>    19
<211>    23
<212>    DNA
<213>    Artificial Sequence

<220>
<223>    C-RAF probe

<400>    19
ccacatggtc agcaccaccc tgc                                               23

<210>    20
<211>    20
<212>    DNA
<213>    Artificial Sequence

<220>
<223>    EGFR forwrad primer

<400>    20
gaaggagctg cccatgagaa                                                   20

<210>    21
<211>    21
<212>    DNA
<213>    Artificial Sequence

<220>
<223>    EGFR reverse primer


<400>    21
gactatgtcc cgccactgga t                                                21


<210>    22
<211>    21
<212>    DNA
<213>    Artificial Sequence

<220>
<223>    EGFR probe


<400>    22
aaatcctgca tggcgccgtg c                                                21


<210>    23
<211>    21
<212>    DNA
<213>    Artificial Sequence

<220>
<223>    mTOR forward primer


<400>    23
aggccgcatt gtctctatca a                                                21


<210>    24
<211>    24
<212>    DNA
<213>    Artificial Sequence

<220>
<223>    mTOR reverse primer


<400>    24
gcagtaaatg caggtagtca tcca                                             24


<210>    25
<211>    22
<212>    DNA
<213>    Artificial Sequence

<220>
<223>    mTOR probe


<400>    25
tgcaatccag ctgtttggcg cc                                               22


<210>    26
<211>    20

<212>    DNA
<213>    Artificial Sequence

<220>
<223>    FGFR1 forward primer


<400>    26
cacgggacat tcaccacatc                                                    20


<210>    27
<211>    19
<212>    DNA
<213>    Artificial Sequence

<220>
<223>    FGFR1 reverse primer


<400>    27
gggtgccatc cacttcaca                                                     19


<210>    28
<211>    30
<212>    DNA
<213>    Artificial Sequence

<220>
<223>    FGFR1 probe


<400>    28
actataaaaa gacaaccaac ggccgactgc                                         30


<210>    29
<211>    19
<212>    DNA
<213>    Artificial Sequence

<220>
<223>    B2M forward primer


<400>    29
cattcgggcc gagatgtct                                                     19


<210>    30
<211>    24
<212>    DNA
<213>    Artificial Sequence

<220>
<223>    B2M reverse primer


<400>    30
ctccaggcca gaaagagaga gtag                                               24

<210>	31
<211>	22
<212>	DNA
<213>	Artificial Sequence

<220>
<223>	B2M probe


<400>	31
ccgtggcctt agctgtgctc gc	22


<210>	32
<211>	21
<212>	DNA
<213>	Artificial Sequence

<220>
<223>	GAPDH forward primer


<400>	32
cacatggcct ccaaggagta a	21


<210>	33
<211>	24
<212>	DNA
<213>	Artificial Sequence

<220>
<223>	GAPDH reverse primer


<400>	33
tgagggtctc tctcttcctc ttgt	24


<210>	34
<211>	22
<212>	DNA
<213>	Artificial Sequence

<220>
<223>	GAPDH probe


<400>	34
ctggaccacc agccccagca ag	22


<210>	35
<211>	20
<212>	DNA
<213>	Artificial Sequence

<220>
<223>	HMBS forward primer


<400>	35
ccagggattt gcctcacctt	20

<210>    36
<211>    21
<212>    DNA
<213>    Artificial Sequence

<220>
<223>    HMBS reverse primer


<400>    36
aaagagatga agcccccaca t                                                21


<210>    37
<211>    26
<212>    DNA
<213>    Artificial Sequence

<220>
<223>    HMBS probe


<400>    37
ccttgatgac tgccttgcct cctcag                                           26


<210>    38
<211>    23
<212>    DNA
<213>    Artificial Sequence

<220>
<223>    HPRT1 forward primer


<400>    38
gctcgagatg tgatgaagga gat                                              23


<210>    39
<211>    21
<212>    DNA
<213>    Artificial Sequence

<220>
<223>    HPRT1 reverse primer


<400>    39
ccagcaggtc agcaaagaat t                                                21


<210>    40
<211>    28
<212>    DNA
<213>    Artificial Sequence

<220>
<223>    HPRT1 probe

<400>    40
ccatcacatt gtagccctct gtgtgctc                                          28


<210>    41
<211>    20
<212>    DNA
<213>    Artificial Sequence

<220>
<223>    SDHA forward primer


<400>    41
cacctagtgg ctgggagctt                                                   20


<210>    42
<211>    24
<212>    DNA
<213>    Artificial Sequence

<220>
<223>    SDHA reverse primer


<400>    42
gcccagtttt atcatctcac aaga                                              24


<210>    43
<211>    27
<212>    DNA
<213>    Artificial Sequence

<220>
<223>    SDHA probe


<400>    43
tggcacttac ctttgtccct tgcttca                                           27